# EUROPEAN PATENT APPLICATION

(11) **EP 1 614 688 A1**
(43) Date of publication of application: **11.01.2006**
(21) Application number: 04727126.7
(22) Date of filing: 13.04.2004
(51) Int. Cl.: C07D 405/12, C07D 411/12, A61K 31/4155, A61K 31/4178, A61P 1/00, A61P 3/10, A61P 9/10, A61P 13/12, A61P 17/02, A61P 25/28, A61P 27/02, A61P 35/00, A61P 39/06

(54) **PHENYLAZOLE COMPOUND, PRODUCTION PROCESS, AND ANTIOXIDANT DRUG**

(30) Priority: 14.04.2003 JP 2003109667; 30.01.2004 JP 2004023032
(71) Applicant: NIPPON SODA CO., LTD., Chiyoda-ku, Tokyo 100-8165 (JP)
(72) Inventor: MOCHIZUKI, Nobuo, Nippon Soda Co., Ltd., Odawara-shi, Kanagawa 250-0280 (JP); UMEDA, Nobuhiro, Nippon Soda Co., Ltd., Odawara-shi, Kanagawa 250-0280 (JP); UCHIDA, Seiichi, Nippon Soda Co., Ltd., Odawara-shi, Kanagawa 250-0280 (JP); IKEYAMA, Seiichi, Nippon Soda Co., Ltd., Odawara-shi, Kanagawa 250-0280 (JP); TSUBOKURA, Shiro, Nippon Soda Co., Ltd., Odawara-shi, Kanagawa 250-0280 (JP); TAKADA, Mitsumasa, Nippon Soda Co., Ltd., Odawara-shi, Kanagawa 250-0280 (JP)
(74) Representative: Wibbelmann, Jobst
(86) International application number: PCT/JP2004/005237
(87) International publication number: WO 2004/092163

(57) **Abstract**

The present invention relates to a compound represented by formula (1): 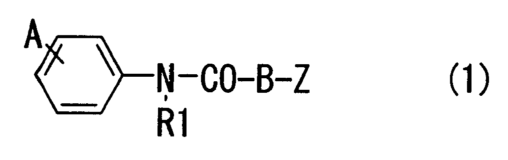 (wherein R represents a hydrogen atom or a C₁₋₆ alkyl group which may be substituted, A represents an imidazolyl group or a pyrazolyl group, B represents a group represented by the following formula: 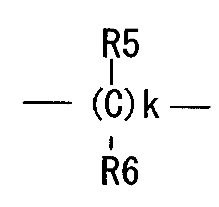 (wherein R5 and R6 each independently represents a hydrogen atom, a cyano group, a hydroxyl group, a halogen atom, a C₁₋₆ alkyl group, a C₁₋₆ alkoxy group, or the like, k represents 0 or an integer of 1 to 15, and R5 and R6 may be identical or different from each other, when k is 2 or more), and Z represents a substituted chroman-2-yl group, a substituted 2,3-dihydrobenzofuran-2-yl, a substituted thiochroman-2-yl group, a substituted 2,3-dihydrobenzothiophene-2-yl group, or a substituted 1,3-benzoxathiol-2-yl group).

## Description

### TECHNICAL FIELD

The present invention relates to a novel phenylazole compound, a production process therefor, an antioxidant having the compound as its active ingredient, and a kidney disease treatment agent, cerebrovascular disease treatment agent, retinal oxidation disorder inhibitor, lipoxygenase inhibitor, and 20-HETE synthase inhibitor, that uses the antioxidant.

### BACKGROUND ART

The formation of lipid peroxides in the body and their associated radical reactions have recently been demonstrated to have various detrimental effects on the body resulting from membrane damage, cytotoxicity and so forth. Accompanying this finding, various attempts have been made to apply antioxidants and lipid peroxide formation inhibitors to pharmaceuticals, and research has been conducted on numerous types of antioxidants (see, for example, Non-Patent Literature 1). Known examples of these antioxidants include pharmaceutical compositions used for the treatment and prevention of endotoxin shock triggered by inflammation or infection that contain a specific quinone derivative (see, for example, Patent Literature 1), hydroxamic acid derivatives used for the treatment and prevention of autoimmune diseases having cell growth inhibitory action and vascularization inhibitory action (see, for example, Patent Literature 2), and 2,3-dihydrobenzofuran derivatives that are useful as antioxidants and radical scavengers (see, for example, Patent Literatures 3, 4 and 5). In addition, other known examples include imidazole-based compounds having anti-hyperlipemia action that are useful for the treatment and prevention of arteriosclerosis (see, for example, Patent Literature 6), and benzothiazine carboxamides represented by the following formula having anti-arthritis activity (see, for example, Patent Literature 7).

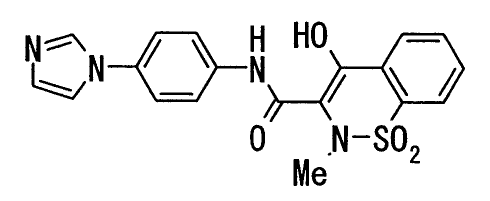

Moreover, other known examples include carbonyl aminophenyl imidazole derivatives (see, for example, Patent Literature 8, Patent Literature 9, and Patent Literature 10), aminodihydrobenzofuran derivatives having lipid peroxide formation inhibitory action that are useful as preventive and treatment agents of various diseases such as arteriosclerosis, liver disease and cerebrovascular disorders (see, for example, Patent Literature 11), anti-hyperlipemia drugs containing phenylazole compounds (see, for example, Patent Literature 12), dihydrobenzofuran derivatives that significantly improve damage caused by lipids, proteins, carbohydrates and DNA occurring as a result of oxidative stress that occurs when antioxidation defense systems are inadequate (see, for example, Patent Literature 13), and optically active aminodihydrobenzofuran derivatives that are effective for improving, treating and preventing impairment of brain function accompanying cerebral stroke or head injury (see, for example, Patent Literature 14).

Since the brain is dependent on circulating blood for its supply of energy despite it having a large energy demand, it is extremely susceptible to ischemia. When the brain falls into a state of cerebral ischemia as a result of having its blood flow cut off for various reasons, active oxygen species are formed triggered by mitochondrial damage and elevated calcium levels within nerve cells. In addition, oxygen radicals are known to be formed in extremely large amounts when blood flow is resumed following ischemia. These active oxygen species ultimately act on lipids, proteins, nucleic acids, or the like, which is said to result in their oxidation and cell death. Antioxidants are used to treat such a condition, and in Japan, Edaravone has been approved as a brain protective drug and is used for that purpose.

Lipoxygenase (abbreviated as LO), which adds oxygen to unsaturated fatty acids such as arachidonic acid, is known to exist in the form of 5-LO, 8-LO, 12-LO and 15-LO according to the site where oxygen is added. Among these, 5-LO is the initial enzyme in the synthesis of leucotrienes, which are potent inflammation mediators. Leucotrienes are involved in various inflammatory diseases such as asthma, rheumatoid arthritis, inflammatory colitis and psoriasis, and their control is useful for the treatment of these diseases. 12-LO and 15-LO react with linoleic acid, cholesterol esters, phospholipids and low-density lipoproteins (hereinafter, abbreviated as LDL) in addition to arachidonic acid, and are known to add oxygen to their unsaturated fatty acids (see Non-Patent Literature 2). Macrophages become foamy cells by unlimited uptake of oxidative-modified LDL by means of scavenger receptors, and this is widely known to be the initial step in the formation of arteriosclerotic foci. 12-LO and 15-LO are represented by high levels in macrophages, and have been clearly demonstrated to be essential as the trigger for oxidative modification of LDL (see Non-Patent Literature 3) . Their control is useful for the treatment of various types of diseases caused by arteriosclerosis (see Patent Literature 15).

20-Hydroxyeicosatetraenoic acid (abbreviated as "20-HETE" hereinafter) is produced by 20-HETE synthase from arachidonic acid, which is a fatty acid precursor separated from phospholipids of the cell membrane. 20-HETE is known to cause dilation and constriction of the microvasculature in major organs such as the kidneys and cerebrovascular system, and to promote cellular proliferation. 20-HETE is involved in major physiologic functions within the body, suggesting its significant participation in pathological states in renal disease, cerebrovascular disease, cardiovascular disease, or the like (Non-Patent Literatures 4 to 6).

Further, phenylazole derivatives (Patent Literatures 16, 17, and 18) have been reported to have an inhibitory effect on 20-HETE synthase.

Oxidative stress involving free radicals and active oxygen is thought to be one of the causative factors of many eye diseases such as cataract and macular degeneration that frequently occurs with aging (see, for example, Non-Patent Literatures 7 to 9). Among tissues of the eye, the retina along with the lens are known to be tissues that are susceptible to the effects of aging (see, for example, Non-Patent Literature 10 ) . The retina is susceptible to the effects of various free radicals because it contains a large amount of higher unsaturated fatty acids and because it is provided with nutrients from both retinal blood vessels and choroid blood vessels and consumes a large amount of oxygen. For example, light such as sunlight that enters the eyes during the entire course of a person's life is a typical example of oxidative stress that affects the retina. The majority of the sunlight that reaches the earth consists of visible light and infrared right, while ultraviolet light that only accounts for several percent of that light has a significant effect on health by powerfully interacting with the body as compared with visible light and infrared light. Ultraviolet light is divided into UV-A (320 to 400 nm), UV-B (280 to 320 nm) and UV-C (190 to 280 nm) according to differences in its wavelength, and although its action and strength relative to the body differ, ultraviolet light of 290 nm or less which exhibits particularly strong cytotoxicity has conventionally been thought to hardly reach the earth's surface at all as a result of being absorbed by the ozone layer of the stratosphere. In recent years, however, the amount of ultraviolet light that reaches the earth has increased due to the appearance of ozone holes thought to be caused by destruction of the environment, and judging from rapid increases in the occurrences of skin disorders and skin cancer related to ultraviolet light in the southern hemisphere, retinopathy is expected to considerably increase due to the effects of UV-A reaching the earth's surface.

Among various eye diseases, age-related macular degeneration is a type of retinopathy associated with a high degree of vision loss. In the US, roughly 10 million people have mild symptoms of this disease, and more than 450,000 have impaired vision brought on by this disorder (see, for example, Non-Patent Literature 11). There is concern over an increased incidence of this disease in Japan as well where the number of elderly in the general population is increasing rapidly. Although there are many aspects of the mechanism of occurrence of macular degeneration that are not fully understood, the progression of this lesion has been pointed out to involve a peroxidation reaction caused by light absorption in the retina (see, for example, Non-Patent Literatures 12 and 13). In addition, the appearance of a lipofuscin-like fluorescent substance known as Druse has been observed in the early stages of its onset. Since lipofuscin is formed from the bonding of protein and aldehyde, which is a secondary decomposition product of lipid peroxides, there is the possibility that a lipid peroxidation reaction in the retina caused by ultraviolet light or visible light may induce this type of retinopathy.

Retina disease treatment agents containing a specific dihydrofuran derivative (see, for example, Patent Literature 19), and drugs for visual acuity and retinal changes, including macular changes of the retina, that contain propionyl L-carnitine or its pharmaceutically acceptable salts, and carotenoids (see, for example, Patent Literature 20) , are known to be useful for the prevention and treatment of these retina diseases due to their antioxidative action.
Patent Literature 1: Japanese Unexamined Patent Application, First Publication No. S61-44840
Patent Literature 2: Japanese Unexamined Patent Application, First Publication No. H1-104033
Patent Literature 3: Japanese Unexamined Patent Application, First Publication No. H2-121975
Patent Literature 4: European Patent Application, Publication No. EU 345593
Patent Literature 5: European Patent Application, Publication No. EU 483772
Patent Literature 6: International Publication No. WO 95/29163
Patent Literature 7: German Patent Application, Publication No. DE 3,407,505
Patent Literature 8: Japanese Unexamined Patent Application, First Publication No. S55-69567
Patent Literature 9: European Patent Application No. EU 324377
Patent Literature 10: European Patent Application, Publication No. EU 458037
Patent Literature 11: Japanese Unexamined Patent Application, First Publication No. H5-140142
Patent Literature 12: International Publication No. WO 00/006550
Patent Literature 13: International Publication No. WO 96/28437
Patent Literature 14: Japanese Unexamined Patent Application, First Publication No. H6-228136
Patent Literature 15: Japanese Unexamined Patent Application, First Publication No. H2-76869
Patent Literature 16: International Publication No. WO 00/0168610
Patent Literature 17: Japanese Unexamined Patent Application, First Publication No. 2004-010513
Patent Literature 18: International Publication No. WO 03/022821
Patent Literature 19: Japanese Unexamined Patent Application, First Publication No. H6-287139
Patent Literature 20 International Publication No. WO 00/07581
Non-Patent Literature 1: Biochem. Biophys. Acta, 1304, 652, 1996; J. Amer. Oil Chemists Soc., 51, 200, 1974.
Non-Patent Literature 2: Biochem. Biophys. Acta, 1304, 652, 1996.
Non-Patent Literature 3: J. Clin. Invest., 103, 15972, 1999.
Non-Patent Literature 4: J. Vascular Research, 32, 79, 1995
Non-Patent Literature 5: Am. J. Phsiol., 277, 607, 1999
Non-Patent Literature 6: Physiol. Rev., 82, 131, 2002
Non-Patent Literature 7: Anderson R.E., Kretzer F.L., Rapp L.M.: "Free Radicals and Eye Diseases", Adv. Exp. Med. Biol., 366, 73, 1994.
Non-Patent Literature 8: Nishigori H., Lee J.W., Yamauchi Y., Iwatsuru M.: "Lipid Peroxide Changes in Glucorticoid-Induced Cataract in Germinated Chicken Embryos and Effects of Ascorbic Acid", Curr. Eye Res., 5, 37, 1986.
Non-Patent Literature 9: Truscott R.J.W., Augusteyn R.C.: "Action of Mercapto Groups in the Normal and Cataract Human Lens", Exp. Eye Res., 25, 139, 1977.
Non-Patent Literature 10: Hiramitsu T., Armstrong D.: "Preventive Effects of Antioxidants Against Lipid Peroxidation Reactions in the Retina", Ophthalmic Research, 23, 196, 1991.
Non-Patent Literature 11: Vitamin Information Center (Tokyo), VIC Newsletter, 105, 4, 2002.
Non-Patent Literature 12: Sachimura S.: "Cataract and Active Oxygen Free Radicals, Active Oxygen Free Radicals", 3, 402, 1992.
Non-Patent Literature 13: Solbach U., Keilhauer C., Knabben H., Wolf S.: "Retina Autofluorescent Images in Age-Related Macular Degeneration", Retina, 17, 385, 1997.

### DISCLOSURE OF THE INVENTION

The object of the present invention is to provide an antioxidant that is effective for the treatment of arteriosclerosis and other ischemic organ disorders such as myocardial infarction and cerebral stroke or for the treatment of diseases caused by oxidative cytotoxicity, and particularly to provide an oxidation disorder inhibitor for the retina that inhibits retinopathy caused by oxidation, and photooxidation in particular, a lipoxygenase inhibitor, and a 20-HETE synthase inhibitor.

As a result of conducting extensive studies to solve the aforementioned problems, the inventors of the present invention determined that the cause of the inadequate effectiveness of existing antioxidants is that the drug either does not reach the target site or ends up losing activity before reaching the target site, and as a result of conducting extensive studies for the purpose of developing an antioxidant that has better organ migration and passes easily through the blood-brain barrier or blood-retina barrier in particular, found that a compound represented by formula (1) achieves the desired effect, and that it has superior in vivo antioxidative action regardless of the administration route, thereby leading to completion of the present invention.

Moreover, the inventors of the present invention also studied effects on the retina by subjecting rat eyes to spot irradiation with a fixed dosage of UV-A. A lipofuscin-like fluorescent substance is frequently detected from the reaction product of proteins and aldehydes originating in lipid peroxides during the early stages of onset of retina diseases having a high degree of vision loss such as macular degeneration. An increase in protein in the vicinity of 66 kDa is observed that is highly proportional to changes in eye retina tissue irradiated with UV-A, and this protein has been observed to be an albumin-like substance based on the results of instrument analyses and studies using albumin-free rats. Since significant increases in a lipofuscin-like fluorescent substance have been observed due to the presence of albumin in automated oxidation reactions of retina tissue in vitro, there is a high probability that abnormal increases in some proteins in retina tissue caused by UV-A irradiation are related to increases in fluorescent substances in the retina, and that this triggers retinopathy. The inventors of the present invention have previously conducted studies on retinopathy inhibitors by using changes in this retina protein as the first biochemical indicator. During the course of those studies, the compound according to the present invention having strong antioxidative action was observed to migrate to the retina in a short period of time following oral administration, and significantly inhibited increases in the 66 kDa protein caused by UV-A spot irradiation. After obtaining findings that the compound according to the present invention is effective against retinopathy caused by oxidation, and particularly effective in diminishing the progress and symptoms of age-related macular degeneration of the retina that increases with age, this result led to completion of the present invention on the basis of those findings.

Namely, the present invention is characterized by the following.
1. A compound represented by formula (1): 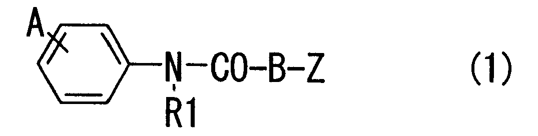 (wherein,
   R1 represents a hydrogen atom or a C₁₋₆ alkyl group which may be substituted,
   A represents an imidazolyl group or a pyrazolyl group represented by the following formulae: 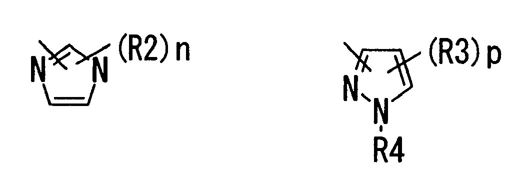
   (wherein
   R2 and R3 represent a hydrogen atom or a C₁₋₆ alkyl group which may be substituted by G1,
   R4 represents a hydrogen atom or a C₁₋₆ alkyl group which may be substituted by G1, a C₁₋₆ alkylcarbonyl group which may be substituted by G1, or a benzoyl group which may be substituted by G1,
   n represents 0 or an integer of 1 to 3,
   p represents 0 or an integer of 1 or 2, and
   R2 and R3 may be identical to each other, or different from each other, when n and p are 2 or more),
   B represents a group represented by the following formula: 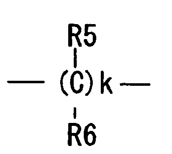 (wherein
   R5 and R6 each independently represents a hydrogen atom, a cyano group, a hydroxyl group, a halogen atom, a C₁₋₆ alkyl group, a C₁₋₆ alkoxy group, a C₂₋₆ alkenyl group, a C₂₋₆ alkynyl group, a C₂₋₆ alkenyloxy group, a C₂₋₆ alkynloxy group, a C₁₋₆ acyloxy group, or a C₃₋₆ cycloalkyl group, or a phenyl group which may have a substituent,
   k represents 0 or an integer of 1 to 15, and
   R5 and R6 may be identical to each other, or different from each other, when k is 2 or more),
   Z represents a chroman-2-yl group which is substituted by G2, a 2, 3-dihydrobenzofuran-2-yl group which is substituted by G2, a thiochroman-2-yl group which is substituted by G2, a 2,3-dihydrobenzothiophene-2-yl group which is substituted by G2, or a 1,3-benzoxathiol-2-yl group which is substituted by G2,
   G1 represents a cyano group, a formyl group, a hydroxyl group, an amino group, a dimethylamino group, or a halogen atom, and
   G2 is represented by the following formula: NHR (wherein R represents a hydrogen atom, a C₁₋₆ alkylcarbonyl group, or a benzoyl group which may have a substituent) or a pharmaceutically acceptable salt thereof.
2. A compound or pharmaceutically acceptable salt as described in 1, wherein z is a group represented by the following formula (A), (B) or (C): 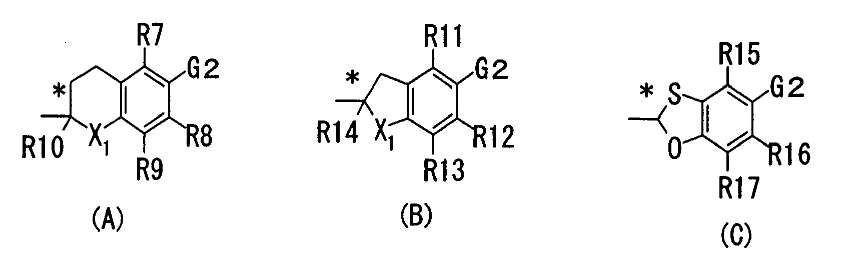 (wherein
   * represents an asymmetric carbon atom,
   X1 represents an oxygen atom or a sulfur atom,
   R7 to R17 each independently represents a hydrogen atom or a C₁₋₆ alkyl group, and
   G2 is represented by the following formula: NHR (wherein R represents a hydrogen atom, a C₁₋₆ alkylcarbonyl group, or a benzoyl group which may have a substituent)).
3. A compound or pharmaceutically acceptable salt as described in 1 or 2, wherein A is 1-imidazolyl or 1-H-pyrazole-5-yl which is substituted at the fourth position on the benzene ring.
4. A production process of a compound represented by formula (1) : 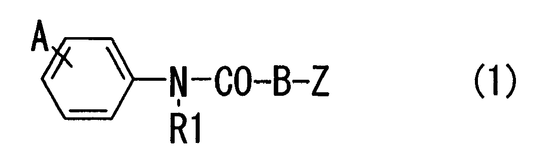 (wherein,
   R1 represents a hydrogen atom or a C₁₋₆ alkyl group which may be substituted,
   A represents an imidazolyl group or a pyrazolyl group represented by the following formulae: 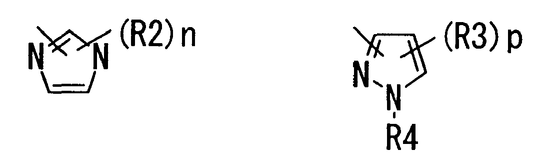
   (wherein
   R2 and R3 represent a hydrogen atom or a C₁₋₆ alkyl group which may be substituted by G1,
   R4 represents a hydrogen atom or a C₁₋₆ alkyl group which may be substituted by G1, a C₁₋₆ alkylcarbonyl group which may be substituted by G1, or a benzoyl group which may be substituted by G1,
   n represents 0 or an integer of 1 to 3,
   p represents 0 or an integer of 1 or 2, and
   R2 and R3 may be identical to each other, or different from each other, when n and p are 2 or more),
   B represents a group represented by the following formula: 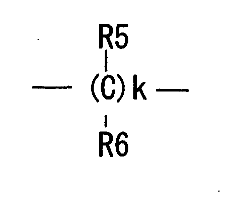 (wherein
   R5 and R6 each independently represents a hydrogen atom, a cyano group, a hydroxyl group, a halogen atom, a C₁₋₆ alkyl group, a C₁₋₆ alkoxy group, a C₂₋₆ alkenyl group, a C₂₋₆ alkynyl group, a C₂₋₆ alkenyloxy group, a C₂₋₆ alkynloxy group, a C₁₋₆ acyloxy group, or a C₃₋₆ cycloalkyl group, or a phenyl group which may have a substituent,
   k represents 0 or an integer of 1 to 15, and
   R5 and R6 may be identical to each other, or different from each other, when k is 2 or more),
   Z represents a chroman-2-yl group which is substituted by G2, a 2, 3-dihydrobenzofuran-2-yl group which is substituted by G2, a thiochroman-2-yl group which is substituted by G2, a 2,3-dihydrobenzothiophene-2-yl group which is substituted by G2, or a 1,3-benzoxathiol-2-yl group which is substituted by G2,
   G1 represents a cyano group, a formyl group, a hydroxyl group, an amino group, a dimethylamino group, or a halogen atom, and
   G2 is represented by the following formula: NHR (wherein R represents a hydrogen atom, a C₁₋₆ alkylcarbonyl group, or a benzoyl group which may have a substituent),
   including:
   a step 1 in which a compound represented by the following formula (1') 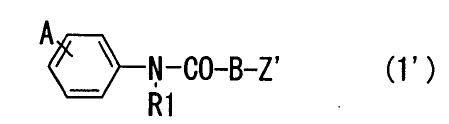 (wherein
      R1 represents a hydrogen atom or a C₁₋₆ alkyl group which may be substituted,
      A represents an imidazolyl group or a pyrazolyl group represented by the following formulae: 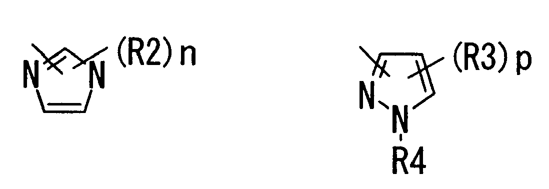 (wherein
      R2 and R3 represent a hydrogen atom or a C₁₋₆ alkyl group which may be substituted by G1,
      R4 represents a hydrogen atom or a C₁₋₆ alkyl group which may be substituted by G1, a C₁₋₆ alkylcarbonyl group which may be substituted by G1, or a benzoyl group which may be substituted by G1,
      n represents 0 or an integer of 1 to 3,
      p represents 0 or an integer of 1 or 2, and
      R2 and R3 may be identical to each other, or different from each other, when n and p are 2 or more)),
      B represents a group represented by the following formula: 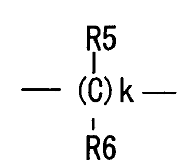 (wherein
      R5 and R6 each independently represents a hydrogen atom, a cyano group, a hydroxyl group, a halogen atom, a C₁₋₆ alkyl group, a C₁₋₆ alkoxy group, a C₂₋₆ alkenyl group, a C₂₋₆ alkynyl group, a C₂₋₆ alkenyloxy group, a C₂₋₆ alkynloxy group, a C₁₋₆ acyloxy group, or a C₃₋₆ cycloalkyl group, or a phenyl group which may have a substituent,
      k represents 0 or an integer of 1 to 15, and
      R5 and R6 may be identical to each other, or different from each other, when k is 2 or more) and
      Z' is represented by the following formula (A)', (B)', or (C)': 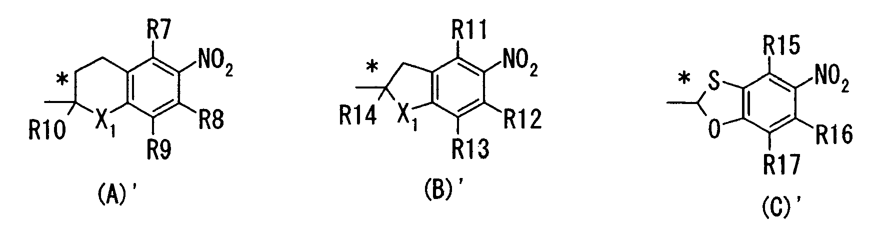
      (wherein
      * represents an asymmetric carbon atom,
      X1 represents an oxygen atom or a sulfur atom,
      R7 to R17 each independently represents a hydrogen atom or a C₁₋₆ alkyl group, and
      G2 is represented by the following formula: NHR (wherein R represents a hydrogen atom, a C₁₋₆ alkylcarbonyl group, or a benzoyl group which may have a substituent)) is produced by reacting an amine compound represented by formula (2) : 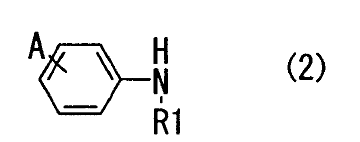 (wherein
      R1 represents a hydrogen atom or a C₁₋₆ alkyl group which may be substituted, and
      A represents an imidazolyl group or a pyrazolyl group represented by the following formulae: 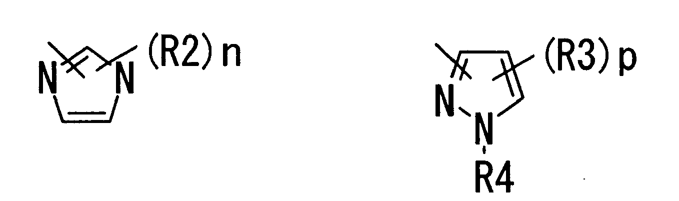 (wherein
      R2 and R3 represent a hydrogen atom or a C₁₋₆ alkyl group which may be substituted by G1,
      R4 represents a hydrogen atom or a C₁₋₆ alkyl group which
      may be substituted by G1, a C₁₋₆ alkylcarbonyl group which may be substituted by G1, or a benzoyl group which may be substituted by G1,
      n represents 0 or an integer of 1 to 3,
      p represents 0 or an integer of 1 or 2, and
      R2 and R3 may be identical to each other, or different from each other, when n and p are 2 or more))
      with a compound represented by the following formula (3):

      YOC-B-Z' (3)

      (wherein
      Y represents a hydroxyl group or a halogen atom,
      B represents a group represented by the following formula: 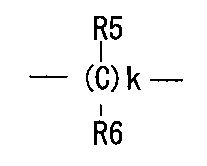 (wherein
      R5 and R6 each independently represents a hydrogen atom, a cyano group, a hydroxyl group, a halogen atom, a C₁₋₆ alkyl group, a C₁₋₆ alkoxy group, a C₂₋₆ alkenyl group, a C₂₋₆ alkynyl group, a C₂₋₆ alkenyloxy group, a C₂₋₆ alkynloxy group, a C₁₋₆ acyloxy group, or a C₃₋₆ cycloalkyl group, or a phenyl group which may have a substituent,
      k represents 0 or an integer of 1 to 15, and
      R5 and R6 may be identical to each other, or different from each other, when k is 2 or more), and
      Z' is represented by the following formula (A)', (B)', or (C)': 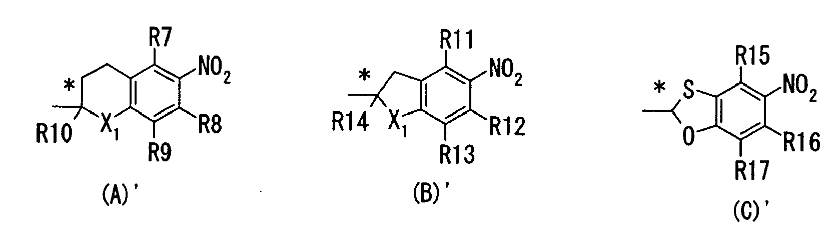
      (wherein
      * represents an asymmetric carbon atom,
      X1 represents an oxygen atom or a sulfur atom,
      R7 to R17 each independently represents a hydrogen atom or a C₁₋₆ alkyl group, and
      G2 is represented by the following formula: NHR (wherein R represents a hydrogen atom, a C₁₋₆ alkylcarbonyl group, or a benzoyl group which may have a substituent)); and
   a step 2 in which the nitro compound produced in the step 1 is converted to an amino group using a reducing agent.
5. An antioxidant that contains as its active ingredient at least one compound represented by formula (1): 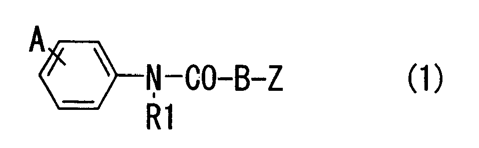 (wherein
   R1 represents a hydrogen atom or a C₁₋₆ alkyl group which may be substituted,
   A represents an imidazolyl group or a pyrazolyl group represented by the following formulae: 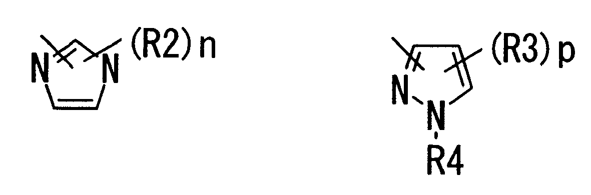 (wherein
   R2 and R3 represent a hydrogen atom or a C₁₋₆ alkyl group which may be substituted by G1,
   R4 represents a hydrogen atom or a C₁₋₆ alkyl group which may be substituted by G1, a C₁₋₆ alkylcarbonyl group which may be substituted by G1, or a benzoyl group which may be substituted by G1,
   n represents 0 or an integer of 1 to 3,
   p represents 0 or an integer of 1 or 2, and
   R2 and R3 may be identical to each other, or different from each other, when n and p are 2 or more)),
   B represents a group represented by the following formula: 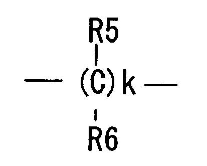 (wherein
   R5 and R6 each independently represents a hydrogen atom, a cyano group, a hydroxyl group, a halogen atom, a C₁₋₆ alkyl group, a C₁₋₆ alkoxy group, a C₂₋₆ alkenyl group, a C₂₋₆ alkynyl group, a C₂₋₆ alkenyloxy group, a C₂₋₆ alkynloxy group, a C₁₋₆ acyloxy group, or a C₃₋₆ cycloalkyl group, or a phenyl group which may have a substituent,
   k represents 0 or an integer of 1 to 15, and
   R5 and R6 may be identical to each other, or different from each other, when k is 2 or more),
   Z represents a chroman-2-yl group which is substituted by G2, a 2,3-dihydrobenzofuran-2-yl group which is substituted by G2, a thiochroman-2-yl group which is substituted by G2, a 2,3-dihydrobenzothiophene-2-yl group which is substituted by G2, or a 1,3-benzoxathiol-2-yl group which is substituted by G2,
   G1 represents a cyano group, a formyl group, a hydroxyl group, an amino group, a dimethylamino group, or a halogen atom, and
   G2 is represented by the following formula: NHR (wherein
   R represents a hydrogen atom, a C₁₋₆ alkylcarbonyl group, or a benzoyl group which may have a substituent) or a pharmaceutically acceptable salt thereof.
6. An antioxidant as described in 5, wherein in formula (1) z is represented by the following formula (A), (B), or (C): 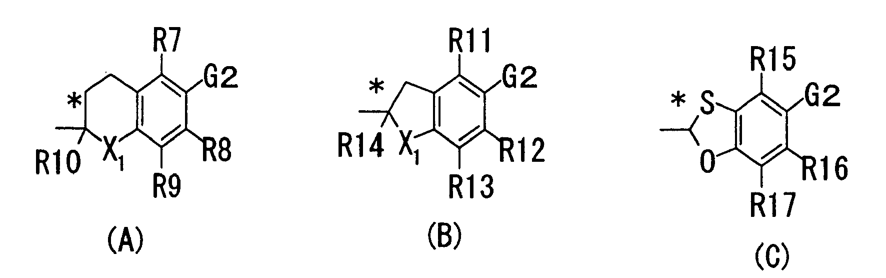 (wherein
   * represents an asymmetric carbon atom,
   X1 represents an oxygen atom or a sulfur atom,
   R7 to R17 each independently represents a hydrogen atom or a C₁₋₆ alkyl group, and
   G2 is represented by the following formula: NHR (wherein R represents a hydrogen atom, a C₁₋₆ alkylcarbonyl group, or a benzoyl group which may have a substituent)).
7. A kidney disease, cerebrovascular or cardiovascular disease treatment agent characterized by containing the antioxidant as described in 6.
8. A cerebral infarction treatment agent characterized by containing the antioxidant as described in 6.
9. A retinal oxidation disorder inhibitor characterized by containing the antioxidant as described in 6.
10. A retinal oxidation disorder inhibitor as described in 9 for age-related macular degeneration or diabetic retinopathy.
11. A lipoxygenase inhibitor characterized by containing the antioxidant as described in 6.
12. A 20-hydroxyeicosatetraenoic acid (20-HETE) synthase inhibitor characterized by containing the antioxidant as described in 6.

### BEST MODE FOR CARRYING OUT THE INVENTION

R1 preferably represents a hydrogen atom or a C₁₋₆ alkyl group such as methyl, ethyl, propyl, isopropyl, butyl, isobutyl, s-butyl, t-butyl or the like in the compound represented by the above-mentioned formula (1). Among these, a hydrogen atom or a methyl group is preferable.

R2 and R3 which are substituents on the imidazolyl group or pyrazolyl group in the definition of A preferably represent a hydrogen atom or a C₁₋₆ alkyl such as methyl, ethyl, propyl, isopropyl, butyl, isobutyl, s-butyl, t-butyl, or the like, that may be substituted by G1.

R4 represents a hydrogen atom; a C₁₋₆ alkyl group such as methyl, ethyl, propyl, isopropyl, butyl, isobutyl, s-butyl, t-butyl, or the like, that may be substituted by G1, a C₁₋₆ alkyl carbonyl group such as acetyl, propionyl, butyryl, isobutyryl, valeryl, pivaloyl or the like, that may be substituted by G1, or a benzoyl group that may be substituted by G1.

When R4 is a hydrogen atom, the pyrazolyl group can take a tautomeric structure shown below.

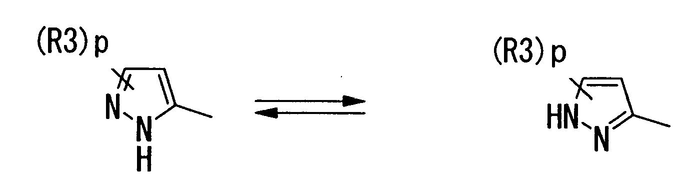

Preferable examples of A include a 1-imidazolyl group, 1H-pyrazol-5-yl group, 1-H-pyrazol-4-yl group, 1-methylpyrazol-5-yl group, 1-methylpyrazol-3-yl group, and 1-benzylpyrazol-4-yl group.

R5 and R6 in the definition of B each independently represents a hydrogen atom; a cyano group; a hydroxyl group; a halogen atom such as chlorine, fluorine, bromine, iodine, or the like; a C₁₋₆ alkyl group such as methyl, ethyl, propyl, isopropyl, butyl, isobutyl, s-butyl, t-butyl or the like; or a C₁₋₆ alkyl group (that may be substituted by a halogen atom such as chlorine, bromine, fluorine, iodine or the like, or a C₁₋₆ alkoxy group such as methoxy, ethoxy, propoxy, isopropoxy or the like) ; a C₁₋₆ alkoxy group such as methoxy, ethoxy, propoxy, isopropoxy, butoxy or the like; a C₂₋₆ alkenyl group such as ethenyl, 1-propenyl, 1-methylvinyl, aryl, 1-methylaryl, 2-butenyl or the like; a C₂₋₆ alkynyl group such as ethynyl, 1-propynyl, 2-propynyl or the like, a C₂₋₆ alkenyloxy group such as ethenyloxy, 1-propenyloxy, 1-methylvinyloxy, aryloxy, 1-methylaryloxy, 2-butenyloxy or the like; a C₂₋₆ alkynyloxy group such as ethynyloxy, 1-propynyloxy, 2-propynyloxy or the like, a C₁₋₆ acyloxy group such as an acetoxy group, propionyloxy group, butyloxy group or the like; a C₃₋₆ cycloalkyl group such as cyclopropyl, cyclobutyl, cyclopentyl, cyclohexyl or the like; or a phenyl group that may have at an optional position on the benzene ring a substituent such as a nitro group; a halogen atom such as chlorine, bromine, fluorine, iodine or the like; a C₁₋₆ alkyl group such as methyl, ethyl, propyl, isopropyl, butyl, isobutyl, s-butyl, t-butyl, or the like; a C₁₋₆ alkoxy group such as methoxy, ethoxy, propoxy, isopropoxy, butoxy or the like; or a C₁₋₆ haloalkyl group such as chloromethyl, dichloromethyl, trichloromethyl, trifluoromethyl, 1-fluoroethyl, 1,1-difluoroethyl, pentafluoroethyl or the like.

k represents 0 or an integer of 1 to 15. When k is 2 or more, then a plurality of R5s and R6s, respectively, may be present, and these multiple R5 and R6 may be identical to each other or different from each other.

R5 and R6 in B are preferably a hydrogen atom, methyl group or phenyl group, and k is preferably 0, 1, 2, 3, 4 or 5.

Examples of Z include the following cyclic groups: a chroman-2-yl group that may have a substituent, a 2,3-dihydrobenzofuran-2-yl group that may have a substituent, a thiochroman-2-yl group that may have a substituent, a 2,3-dihydrobenzothiophene-2-yl group that may have a substituent, or a 1, 3-benzoxathiol-2-yl group that may have a substituent.

Groups represented by the following various structural formulae may be cited for the aforementioned Z.

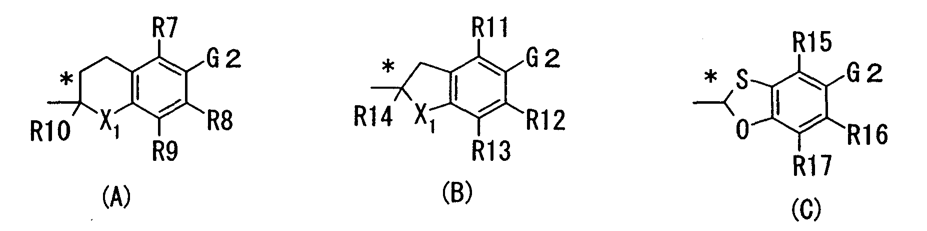

In the formulae, *, X1 and q have the same meaning as disclosed above.

R7, R8, R9, R10, R11, R12, R13, R14, R15, R16 and R17 each independently represents a hydrogen atom; or a C₁₋₆ alkyl group such as methyl, ethyl, propyl, isopropyl, butyl, isobutyl, s-butyl, t-butyl or the like.

G1 represents a cyano group; a formyl group; a hydroxyl group; an amino group; a dimethylamino group; or a halogen atom such as chlorine, bromine, fluorine, iodine, or the like.

G2 is represented by the following formula: NHR (wherein, R indicates a hydrogen atom; a C₁₋₆ alkylcarbonyl group such as acetyl, propionyl, butyryl, isobutyryl, valeryl, pivaloyl or the like; or a benzoyl group that may have a substituent such as a nitro group; a halogen atom such as chlorine, bromine, fluorine, iodine or the like; a C₁₋₆ alkyl group such as methyl, ethyl, propyl, isopropyl, butyl, isobutyl, s-butyl, t-butyl or the like; a C₁₋₆ alkoxy group such as methoxy, ethoxy, propoxy, isopropoxy, butoxy or the like; a C₁₋₆ haloalkyl group such as chloromethyl, dichloromethyl, trichloromethyl, trifluoromethyl, 1-fluoroethyl, 1,1-difluoroethyl, pentafluoroethyl or the like).

### (Production process for the compounds)

The compounds represented by the above formula (1) according to the present invention can be produced by the following process, for example.

### Production process 1

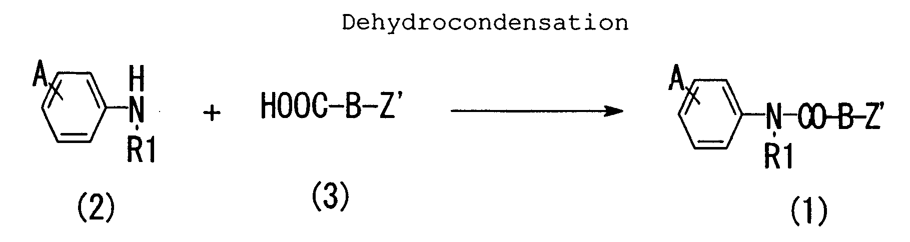

(In the formulae, A, B, R1 and Z' have the same meaning as disclosed above.)

Namely, the carboxylic acid represented by formula (3) and the amine represented by formula (2) are subjected to dehydrocondensation by a common method, to obtain the amide derivative represented by formula (1), which is the compound according to the present invention.

This dehydrocondensation reaction can be carried out in the presence of a suitable condensing agent. Examples of the condensing agent include 1,3-dicyclohexylcarbodiimide, 1-(3-dimethylaminopropy)-3-ethylcarbodiimide, 2-ethoxy-1-ethoxycarbonyl-1,2-dihydroquinoline, and the like.

Further, the reaction can be carried out even faster by reacting the reaction system in the presence of N-hydroxysuccinimide, 1-hydroxybenzotriazole, or 3,4-dihydro-3-hydroxy-4-oxo-1,2,3-benzotriazine.

The reaction solvent is not particularly limited provided that it is an inert solvent in the reaction. Examples thereof include ethers such as diethylether, tetrahydrofuran (THF), 1,4-dioxane, and the like, aromatic hydrocarbons such as benzene, toluene, xylene and the like, halogenated hydrocarbons such as dichloromethane, chloroform, 1,2-dichloroethane and the like, acetonitrile, dimethylformamide (abbreviated as DMF hereinafter), dimethylsulfoxide (abbreviated as DMSO hereinafter), pyridine and the like.

The reaction is carried out in the range of -15°C to roughly the boiling point of the solvent, and preferably in the range of 0 to 80°C.

### Production process 2

As another process, the compound can also be produced according to the following reaction formula. 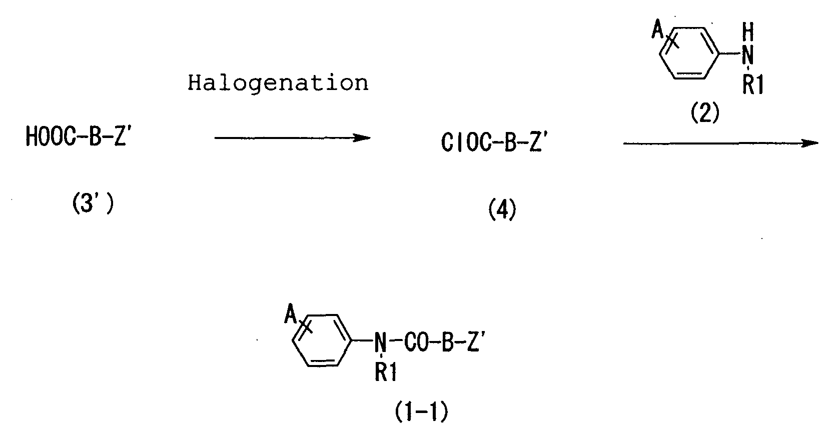 (In the formulae, A, B, R1 and Z' have the same meaning as described above.)

Namely, the carboxylic acid derivative represented by formula (3') is reacted with a halogenating agent such as thionyl chloride, phosphorus pentachloride, oxalyl chloride or the like, to obtain the acid chloride (4) . Next, the obtained acid chloride is reacted with the amine represented by formula (2) in an inert organic solvent and in the presence of a base.

The reaction solvent is not particularly limited provided that it is an inert solvent in the reaction. Examples thereof include ethers such as diethylether, THF, 1,4-dioxane, or the like, aromatic hydrocarbons such as benzene, toluene, xylene, or the like, halogenated hydrocarbons such as dichloromethane, chloroform, 1, 2-dichloroethane or the like, acetonitrile, DMF, DMSO, pyridine or the like.

Examples of the base that may be used in the reaction include amines such as triethylamine, pyridine, 1,8-diazabicyclo[5,4.0]undec-7-ene (abbreviated as DBU hereinafter),and the like, and inorganic salts such as sodium hydrogencarbonate, sodium carbonate, potassium carbonate, sodium hydroxide, and the like.

The reaction is carried out in the range of -15°C to roughly the boiling point of the solvent, and preferably in the range of 0 to 80°C.

### Production process 3

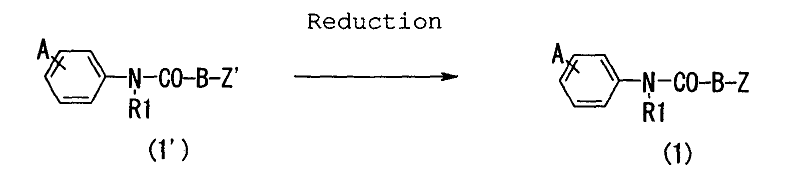

(In the formulae, A, B, R1, Z and Z' have the same meaning as described above.)

Namely, by carrying out a hydrogen addition to the nitro compound represented by formula (1') using a catalyst, the aniline compound represented by formula (1) can be obtained.

Examples of catalysts include palladium carbon, platinum dioxide, Raney nickel, and the like.

Examples of the solvent include alcohols such as methanol and ethanol, ethers such as diethyl ether, THF and 1,4-dioxane, hydrocarbons such as benzene, toluene, xylene and cyclohexane, amides such as DMF, organic acids such as formic acid and acetic acid, esters such as ethyl acetate and mixed solvents thereof.

The reaction is carried out at 0°C to roughly the boiling point of the solvent, and is preferably carried out at 20°C to 80°C.

Target compounds can be produced by carrying out conventional post-treatments after the end of the reaction.

The structure of the compound of the present invention is determined by IR, NMR and MS.

Furthermore, the compound represented by the aforementioned formula (1) and the raw material compounds represented by the aforementioned formulae (3) and (4) can also have several optically active substances and tautomers. These are all included in the scope of the present invention.

Examples of pharmaceutically acceptable salts of the compound represented by the aforementioned formula (1) include salts of inorganic acids such as hydrochloric acid, sulfuric acid, nitric acid and phosphoric acid, and salts of organic acids such as acetic acid, propionic acid, lactic acid, succinic acid, tartaric acid, citric acid, benzoic acid, salicylic acid, nicotinic acid and heptagluconic acid. These can be easily produced by ordinary chemical synthesis methods.

### (Antioxidant)

Since the phenylazole compound of the present invention has antioxidative action, it can prevent the onset and progress of arteriosclerotic lesions by preventing oxidative degeneration of low density lipoproteins (hereinafter, abbreviated as LDL), so it can be applied to treatment agents for arteriosclerosis. Also, it is useful as a treatment agent for various diseases such as senile dementia-related diseases, heart disease, cancer, diabetes, gastrointestinal diseases, burns, eye diseases and kidney disease that are based on oxidative action. Moreover, although various types of active oxygen are generated when blood is reperfused at ischemic sites, and tissue damage is exacerbated due to cell membrane damage caused by lipid peroxidation in ischemic organ diseases such as cerebral stroke and myocardial infarction, the phenylazole compound of the present invention is able to prevent tissue damage at sites of ischemic lesions by removing the various types of active oxygen and lipid peroxides due to its antioxidative activity, so it can be applied to treatment agents for ischemic organ disorders. In addition, since the phenylazole compound of the present invention has lipoxygenase inhibitory action and 20-HETE synthase inhibitory action, it can inhibit the conversion of arachidonic acid to HPETE by inhibiting the action of lipoxygenase and the production of 20-HETE by inhibiting 20-HETE synthase. Moreover, some of the compounds according to the present invention has a low dopamine-release inhibitory action, which results in low possibility of causing parkinsonian side effect or the like.

Moreover, the phenylazole compound of the present invention can be used for the prevention and treatment of diseases caused by oxidative disorders of the retina, diabetes, hypertension, arteriosclerosis, anemia, leukemia, connective tissue diseases such as systemic lupus erythematosus or sclerosis, vascular disorders, inflammatory, or degenerative lesions of the retina caused by systemic diseases such as congenital metabolic abnormalities such as Tay-Sacks disease or Vogt-Spielmeyer disease, disorders of retinal blood vessels such as retinopathy of immaturity, retinal vein occlusion, retinal artery occlusion or retinal periphlebitis, inflammation or degeneration of the retina caused by retina detachment or injury, degenerative diseases of the retina accompanying aging such as age-related macular degeneration, or local retina diseases such as congenital retina degenerative diseases, and is particularly useful as a treatment agent for diseases such as age-related macular degeneration that occur due to photooxidative disorders.

The antioxidant according to the present invention is not particularly limited, provided that it contains as its active ingredient at least one selected from the phenylazole compounds according to the present invention having the aforementioned antioxidative action and the pharmaceutically acceptable salts thereof, and it can be administered as a drug for the aforementioned diseases by any arbitrary route. Examples of the administration route include oral, transnasal, parenteral, local, transcutaneous or transrectal administration. Its drug form may be a suitable drug form such as a solid, semi-solid, freeze-dried powder or liquid, specific examples of which include tablets, suppositories, pills, soft and hard capsules, powders, liquids, injection preparations, suspensions, aerosols and sustained-release preparations, which allow an accurate dosage to be formulated and administered easily.

In addition, an antioxidant of the present invention can be in the form of a composition that contains in addition to an active ingredient and commonly used pharmaceutical carriers or vehicles, other drugs, adjuvants and so forth within a range that does not react with other ingredients. This composition can be made to contain the active ingredient at 1 to 99% by weight and a suitable pharmaceutical carrier or vehicle at 99 to 1% by weight corresponding to the administration route, and preferably to contain 5 to 75% by weight of the active ingredient and a suitable pharmaceutical carrier or vehicle as the remainder.

In the antioxidant according to the present invention, a small amount of an auxiliary substance such as a lubricant, emulsifier, pH buffer, antioxidant or the like may be contained within a range that does react with the other ingredients as desired regardless of the administration route thereof, examples of which include citric acid, sorbitan monolaurate, triethanol amine oleate and butylated hydroxytoluene.

This type of preparation can be produced according to the conventional method, for example, the description taught in Remington's Pharmaceutical Sciences, Volume 18, Mack Publishing Company, Easton, Pennsylvania, 1990.

In the antioxidant of the present invention, the therapeutically effective amount of the compound represented by formula (1) or the pharmaceutically acceptable salt thereof varies according to the individual and the symptoms of the disease being treated. Normally, the daily therapeutically effective dosage may be 0.14 mg to 14.3 mg/day of at least one compound represented by formula (1) or pharmaceutically acceptable salt thereof per 1 kg of body weight, preferably 0. 7 mg to 10 mg/day per 1 kg of body weight, and more preferably 1.4 mg to 7.2 mg/day per 1 kg of body weight. For example, in the case of administering to a human having a body weight of 70 kg, the dosage range of the compound of formula (1) or the pharmaceutically acceptable salt thereof is 10 mg to 1.0 g per day, preferably 50 mg to 700 mg per day, and more preferably 100 mg to 500 mg per day. However, this dosage range is meant to serve only as a reference, and the dosage may be made to be outside these ranges according to the symptoms being treated.

Examples of the vehicles contained in the antioxidant for oral administration according to the present invention include any normally used vehicles such as pharmaceutical mannitol, lactose, starch, gelatinized starch, magnesium stearate, sodium saccharine, talc, cellulose ether derivatives, glucose, gelatin, sucrose, citrates and propyl gallate. In addition, the antioxidant for oral administration may also contain a diluent such as lactose, sucrose or dicalcium phosphate, a disintegration agent such as cross carmellose sodium or derivatives thereof, a binder such as magnesium stearate, or a lubricant such as starch, gum arabic, polyvinyl pyrrolidone, gelatin or cellulose ether derivatives.

In the case of using as an injection preparation, sterile, aqueous and non-aqueous solutions, suspensions or emulsions may be contained. Examples of diluents of aqueous solutions and suspensions include distilled water for injection and physiological saline. Examples of diluents of non-aqueous solutions and suspensions include propylene glycol, polyethylene glycol, vegetable oils such as olive oil, alcohols such as ethanol, and Polysorbate (trade name). This type of composition may also additionally contain an additive such as an isotonic agent, antiseptic, lubricant, emulsifier, dispersant, stabilizer (for example, lactose) and solubilization or solubility adjuvant. These can also be used by filtering by passing through a bacteria-retaining filter, producing a solid composition of a disinfectant, and dissolving in sterile water or sterile injection solvent prior to use.

In addition, in the case of using the antioxidant of the present invention in the form of a suppository, a carrier that gradually dissolves in the body such as polyoxyethylene glycol or polyethylene glycol (hereinafter, abbreviated as PEG), specific examples of which include PEG1000 (96%) and PEG4000 (4%), may be used, and an example of such a suppository has 0.5 to 50% by weight of the compound of formula (1) or the pharmaceutically acceptable salt thereof dispersed in the carrier.

In the case of using the antioxidant of the present invention in the form of a liquid, water, saline solution, aqueous dextrose solution, glycerol, ethanol or the like is used as a carrier, and the liquid is preferably in the form of a solution or suspension obtained by treatment such as dissolving or dispersing 0.5 to 50% by weight of the compound of formula (1) or the pharmaceutically acceptable salt thereof along with an arbitrary pharmaceutical adjuvant in the carrier.

### (Retina Photooxidative Disorder Inhibitor)

There are no particular limitations on a retina photooxidative disorder inhibitor of the present invention provided that it contains as its active ingredient one or more phenylazole compounds of the present invention and pharmaceutically acceptable salts thereof having the aforementioned antioxidative action, and the administration route, administration form and dosage can be the same as the route, form and dosage of the aforementioned antioxidant. In addition, it may also contain the same preparation ingredients, carriers and adjuvants, etc. as the aforementioned antioxidant, one or more types of vehicles, disintegration agents or binders, etc. as well as other retina oxidative disorder inhibitors that do not react with the active ingredient may be suitably added, and ingredients having other pharmacological effects may be suitably contained in addition to the aforementioned ingredients. In addition, the administration form can also be in the form of an ophthalmic solution or ophthalmic ointment in addition to the same administration forms as those of the aforementioned antioxidant.

In the case of using a retina photooxidative disorder inhibitor of the present invention in the form of an ophthalmic solution, it can be obtained in the form of an aqueous solution or suspension by adding the phenylazole compound of the present invention to a normally used base solvent, and adjusting the pH to 4 to 10 and preferably to 5 to 9. The ophthalmic solution is preferably subjected to sterilization treatment to obtain a sterile product, and the sterilization treatment can be carried out at any stage of the production process. The concentration of the phenylazole compound of the present invention in an ophthalmic solution is 0.001 to 3% (W/V) and preferably 0.01 to 1% (W/V) , and the dosage can be several drops each one to four times per day according to the degree of the symptoms, patient status and various other conditions. The aforementioned dosage is meant to serve only as a reference, and the dosage can also be increased beyond these ranges.

Various types of additives such as a buffer, isotonic agent, antiseptic, pH adjuster, thickener, chelating agent or solubilization agent may be suitably added to the aforementioned ophthalmic solution within a range that does not react with a phenylazole compound of the present invention. Examples of the buffers include citrate buffers, tartrate buffers, acetate buffers and amino acids. Examples of isotonic agents include sugars such as sorbitol, glucose and mannitol, polyvalent alcohols such as glycerin, polyethylene glycol and propylene glycol, and salts such as sodium chloride. Examples of antiseptics include paraoxybenzoic acid esters such as methyl paraoxybenzoate and ethyl paraoxybenzoate, benzyl alcohol, phenethyl alcohol, sorbic acid and salts thereof. Examples of pH adjusters include phosphoric acid and sodium hydroxide. Examples of thickeners include hydroxyethyl cellulose, hydroxypropyl cellulose, methyl cellulose, hydroxypropyl methyl cellulose, carboxymethyl cellulose and salts thereof. Examples of chelating agents include sodium edetate, sodium citrate and condensed sodium phosphate. Examples of solubilization agents include ethanol and polyoxyethylene hardened castor oil.

In addition, in the case of using the retina photooxidative disorder inhibitor of the present invention in the form of an ophthalmic ointment, the phenylazole compound of the present invention may be mixed with a normally used ointment base such as purified lanolin, white Vaseline, Macrogol, Plastibase and liquid paraffin, and it is preferably subjected to sterilization treatment to obtain a sterile product. The concentration of the phenylazole compound of the present invention in the ophthalmic ointment is normally 0.001 to 3% (W/V) and preferably 0.01 to 1% (W/V), and the number of administrations may be one to four times per day according to the degree of the symptoms, patient status and various other conditions. The aforementioned number of administrations is meant to serve only as a reference, and the number of administrations can also be increased beyond these ranges.

Since a retina photooxidative disorder inhibitor of the present invention has superior antioxidative action, it is effective for the prevention and treatment of degenerative diseases of the retina accompanying aging such as age-related macular degeneration.

Although the following provides a detailed explanation of the phenylazole compound of the present invention through its examples, the technical scope of the present invention is not limited to these examples.

### Example 1

### Step 1: Production of (±)-(5-nitro-2, 4, 6, 7-tetramethyldihydrobenzofuran-2-yl)-N-[4-(imidazol-1-yl) phenyl] carboxamide

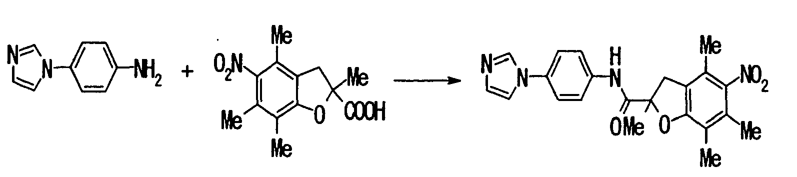

1.06 g of (±)-2, 4, 6, 7-tetramethyl-5-nitrodihydrobenzofuran-2-carboxylic acid, 0.64 g of 1-(4-aminophenyl)imidazole, 0.85 g of 1-(3-dimethylaminopropyl)ethylcarbodiimide hydrochloride, 0.68 g of 1-hydroxybenzotriazole, and 0.7 ml of triethylamine were added to 8 ml of DMF and stirred for 3 hours at 60°C. After cooling, the reaction mixture was poured into ice-water, and the separated product was then extracted with chloroform. The organic layer was washed with brine, dried over anhydrous magnesium sulfate, and concentrated under reduced pressure. The residue was purified by silica gel column chromatography (chloroform : methanol = 100 : 3), to obtain 1. 01 g of the target compound.

### Step 2: Production of (±)-(5-amino-2, 4, 6, 7-tetramethyldihydrobenzofuran-2-yl)-N-[4-(imidazol-1-yl) phenyl] carboxamide

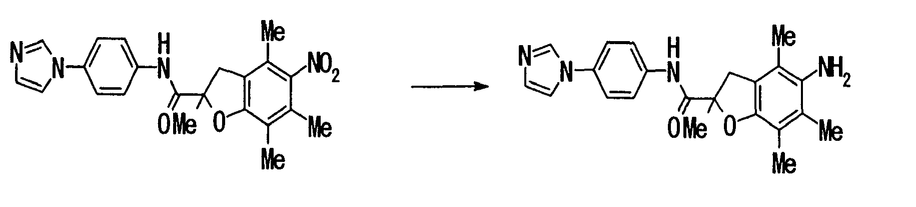

1.01 g of (±) - (5-nitro-2, 4, 6, 7-tetramethyldihydrobenzofuran-2-yl)-N-[4-(imidazol-1-yl) phenyl] carboxamide, 0.5 g of 10% palladium carbon, and 15 ml of methanol were added to an autoclave and stirred overnight under a hydrogen pressure of 5 kg/cm². The reaction mixture was filtered through Celite, and concentrated under reduced pressure. The residue was purified by silica gel column chromatography (chloroform : methanol = 100 : 3) , to obtain 0.33 g of the target compound. The melting point was 160 to 163°C.

### Reference Example 1

### Production of 2, 3, 5-trimethylphenyl 2-methylpropenyl ether

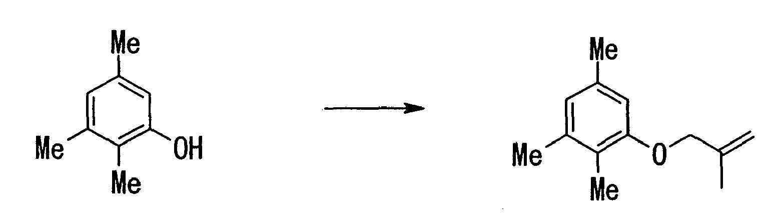

91.1 g of 2, 3, 5-trimethylphenol, 65.3 g of 3-chloro-2-methylpropene, and 99 g of potassium carbonate were added to 700 ml of DMF, and stirred for 3 hours at 80°C. After cooling, the reaction mixture was poured into ice-water, and extracted with ethyl acetate. The extract was washed with water and brine, dried with anhydrous magnesium sulfate, and concentrated under reduced pressure. The residue was purified by silica gel column chromatography (benzene : hexane = 1:1), to obtain 102 g of the target compound.

### Reference Example 2

### Production of 2-aryl-3, 5, 6-trimethylphenol

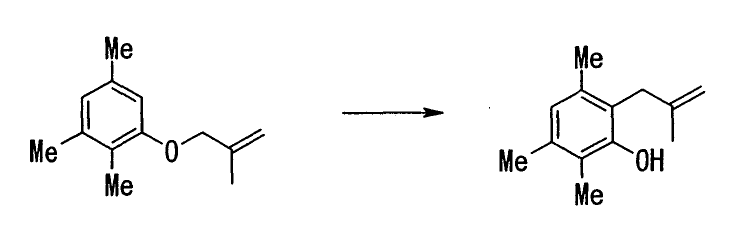

26.6 g of 2, 3, 5-trimethylphenyl 2-methylpropenyl ether was dissolved in 131 ml of diethylaniline, and stirred at 200°C under an argon atmosphere for 2 hours and cooled. The reaction mixture was poured into 6N-hydrochloric acid and extracted with ether. The extract was washed with dilute hydrochloric acid, water, and brine, dried with anhydrous magnesium sulfate, and concentrated under reduced pressure. The residue was purified by silica gel column chromatography (benzene : hexane = 1 : 1) , to obtain 21.4 g of the target compound.

### Reference Example 3

### Production of 2-hydroxymethyl-2, 4, 6, 7-tetramethyldihydrobenzofuran

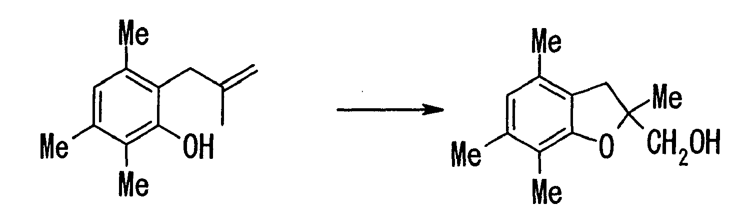

To 31.86 g of 2-aryl-3, 5, 6-trimethylphenol which was dissolved in 600 ml of methylene chloride, 47.5 g of m-chloroperoxybenzoic acid was gradually added at 0°C. The mixture was stirred at 0°C for 2 hours, and poured into aqueous sodium hydrogencarbonate. The organic layer was extracted with chloroform, washed with aqueous sodium hydrogencarbonate, dried with anhydrous magnesium sulfate, and concentrated under reduced pressure. The residue was purified by silica gel column chromatography (chloroform), to obtain 17 g of the target compound.

### Reference Example 4

### Production of 2-hydroxymethyl-2, 4, 6, 7-tetramethyl-5-nitrodihydrobenzofuran

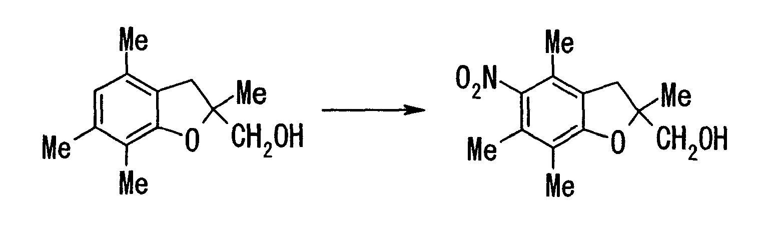

To 2.3 g 2-hydroxymethyl-2,4,6, 7-tetramethyldihydrobenzofuran which was dissolved in 30 ml of acetic anhydride, 1.9 ml of nitric acid was added dropwise at 0°C. After stirring for 1 hour at 0°C, the mixture was poured into ice-water, and stirred at room temperature for 1 hour. The reaction mixture was extracted with ether, washed with brine, dried with anhydrous magnesium sulfate, and concentrated under reduced pressure. The residue was purified by silica gel column chromatography (chloroform), to obtain 1.34 g of the target substance.

### Reference Example 5

### Production of 2, 4, 6, 7-tetramethyl-5-nitrodihydrobenzofuran-2-aldehyde

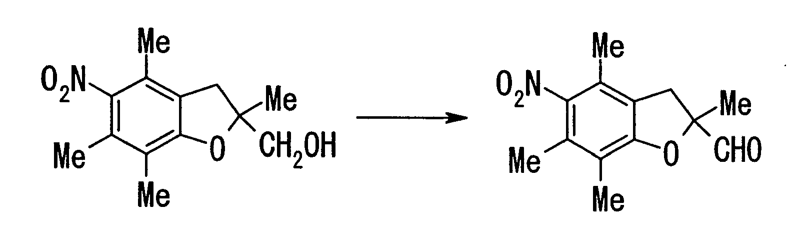

0.57 ml of oxalyl chloride was dissolved in 12 ml of methylene chloride under an argon atmosphere, and cooled to -78°C. A solution of DMSO (1.1 ml) in methylene chloride (2 ml) was added dropwise below -65°C, and stirred for 10 minutes. Then, a solution of 2-hydroxymethyl-2, 4, 6, 7-tetramethyl-5-nitrodihydrobenzofuran (1.34 g) in methylene chloride (4 ml) was added dropwise to the mixture, and stirred for 3 hours at -78°C. After completion of the reaction, 4.2 ml of triethylamine was added dropwise and the mixture was then warmed to room temperature, and 1-N hydrochloric acid was added to the mixture. The reaction mixture was extracted with chloroform, washed with brine, dried with anhydrous magnesium sulfate, and concentrated under reduced pressure. The residue was purified by silica gel column chromatography (chloroform), to obtain 0.86 g of the target compound.

### Reference Example 6

### Production of 2, 4, 6, 7-tetramethyl-5-nitrodihydrobenzofuran-2-carboxylic acid

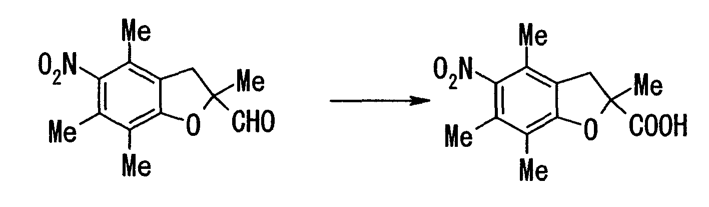

To a solution of 2, 4, 6, 7-tetramethyl-5-nitrodihydrobenzofuran-2-aldehyde (2.39 g) and 2-methyl-2-butene (31 g) in t-butanol (190 ml), a solution of sodium chlorite (7.77 g) and sodium dihydrogen phosphate (10.1 g) in water (78 ml) was added dropwise under ice cooling, and stirred for 2 hours at room temperature. After 2-methyl-2-butene and t-butanol were distilled off under reduced pressure, and water was added to the residue, the mixture was extracted with ether. The extract was washed with brine, dried with anhydrous magnesium sulfate, and concentrated under reduced pressure. Ether-hexane was then added to the residue, the crystals precipitated were filtered off to obtain 1.20 g of the target compound.

### Reference Example 7

### Production of 6-nitro-2-methoxymethyl-2, 5, 7, 8-tetramethylchroman-4-one

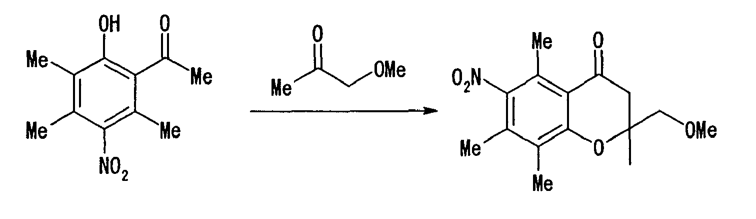

To a solution of 5-nitro-2-hydroxy-3, 4, 6-trimethylacetophenone (66.5 g) and methoxyacetone (78.8 g) in toluene (500 ml), 6.4 g of pyrrolidine was added at room temperature, stirred for 24 hours at room temperature, and refluxed for 3 hours. The reaction mixture was concentrated, and the residue was purified by silica gel column chromatography (hexane : ethyl acetate = 7:1 → 3:1) to obtain 29.2 g of the target compound.

### Reference Example 8

### Production of 6-nitro-4-hydroxy-2-methoxymethyl-2, 5, 7, 8-tetramethylchroman

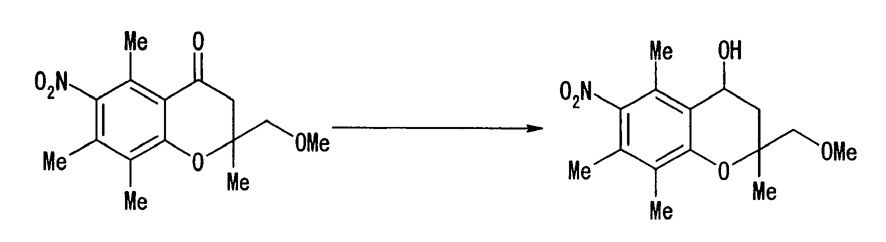

To a solution of 6-nitro-2-methoxymethyl-2, 5, 7, 8-tetramethylchroman-4-one (10 g) in methanol (100 ml) , sodium borohydride (1.3 g) was added at 0°C, and stirred for 1 hour at 0°C. The reaction mixture was then poured into water, and extracted with ethyl acetate. The organic layer was washed with brine, dried with anhydrous magnesium sulfate, and the solvent was evaporated to obtain 10.1 g of the target compound.

### Reference Example 9

### Production of 6-nitro-2-methoxymethyl-2, 5, 7, 8- tetramethyl (2H) chroman

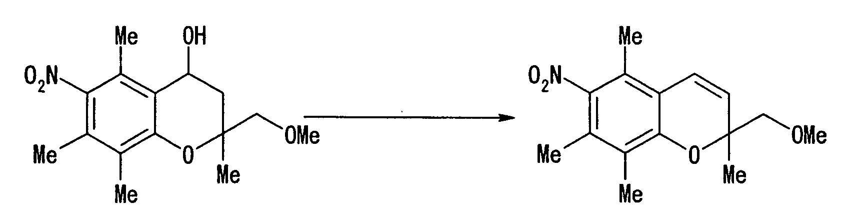

To a solution of 6-nitro-4-hydroxy-2-methoxymethyl-2, 5, 7, 8-tetramethylchroman (10.1g) in benzene (200ml), p-toluene sulfonic acid (1.0 g) was added. The mixture was refluxed using a Dean-Stark apparatus for 2 hours, poured into water, and extracted with ethyl acetate. The organic layer was washed with an aqueous solution of saturated sodium hydrogencarbonate, washed with brine, and dried with anhydrous magnesium sulfate. After magnesium sulfate was removed by filtering, the filtrate was concentrated under reduced pressure to obtain 9.4 g of the target compound in oil form.

### Reference Example 10

### Production of 6-nitro-2-methoxymethyl-2, 5, 7, 8-tetramethylchroman

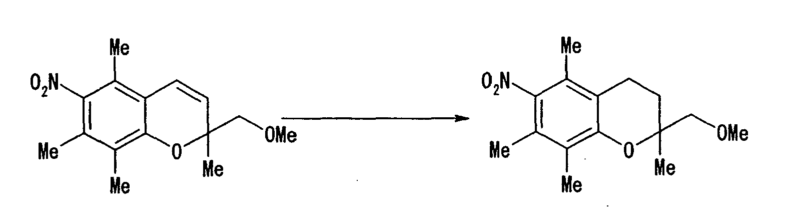

To 9.4 g of 6-nitro-2-methoxymethyl-2, 5, 7, 8- tetramethyl (2H) chroman which was dissolved in 100 ml of ethanol, 1.0 g of 10% palladium charcoal was added. Catalytic hydrogenation was carried out under a hydrogen pressure of 1 atm for 24 hours at room temperature. After the reaction was completed, the reaction mixture was filtered and concentrated under reduced pressure to obtain 9.5 g of the target compound in oil form.

### Reference Example 11

### Production of 6-nitro-2-hydroxymethyl-2, 5, 7, 8-tetramethyl chroman

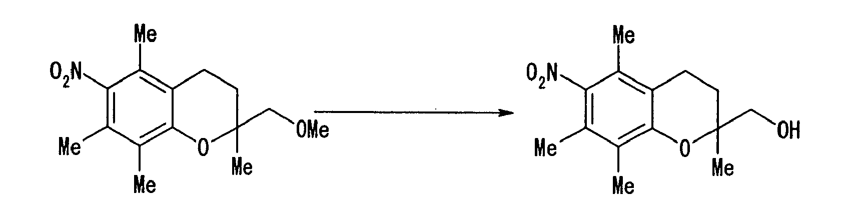

To 9.5 g of 6-nitro-2-methoxymethyl-2, 5, 7, 8-tetramethyl chroman which was dissolved in 80 ml of methylene chloride, 31.4 ml of a 1M boron tribromide solution in methylene chloride was added at 0°C under a nitrogen gas stream, and stirred for 3 hours at 0°C. After completion of the reaction, the reaction mixture was poured into water, and extracted with chloroform. The organic layer was washed with brine, and dried with anhydrous magnesium sulfate. The solvent was concentrated under reduced pressure. The residue was purified by silica gel column chromatography (hexane : ethyl acetate = 2:1), to obtain 4.5 g of the target compound.

### Reference Example 12

### Production of 6-nitro-2-formyl-2, 5, 7, 8-tetramethyl chroman

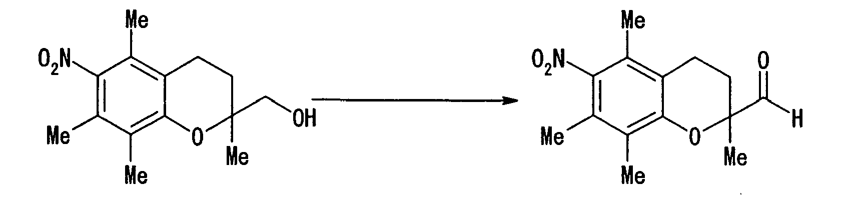

To a solution of oxalyl chloride (1.6 ml) in methylene chloride (40 ml) at -60°C under a nitrogen gas stream, 3.1 ml of DMSO was added dropwise at -60°C, and stirred for 5 minutes. A solution of 3.9 g of 6-nitro-2-hydroxymethyl-2, 5, 7, 8-tetramethyl chroman in 10 ml of methylene chloride was added dropwise under a nitrogen gas stream at -60°C, and then stirred for 30 minutes at -60°C. Next, 12 ml of triethylamine was added at -60°C, and warmed gradually to room temperature to complete the reaction. After completion of the reaction, the reaction mixture was poured into water, and extracted with chloroform. The organic layer was washed with brine, and dried with anhydrous magnesium sulfate. The solvent was concentrated under reduced pressure. The residue was purified by silica gel column chromatography (hexane : ethyl acetate = 2 : 1), to obtain 3.4 g of the target compound in crystal form.

### Reference Example 13

### Production of 6-nitro-2, 5, 7, 8-tetramethyl chroman-2-carboxylic acid

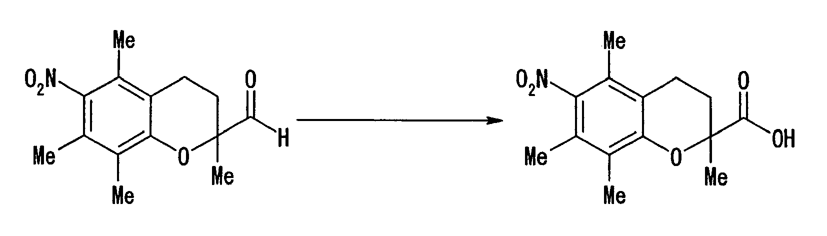

To a solution of 6-nitro-2-formyl-2, 5, 7, 8-tetramethyl chroman (2.3 g) in t-butanol (150 ml), 23 g of 2-methyl-2-butene was added at room temperature. A solution of sodium chlorite (5.8 g) and sodium dihydrogen phosphate dihydrate (7.6 g) in water (60 ml) was added dropwise at room temperature, and stirred for 2 hours at room temperature. After completion of the reaction, the reaction mixture was poured into water, and extracted with ether. 5% sodium hydrogencarbonate was added to the mixture, and the ether layer was discarded. The pH of the aqueous layer was adjusted to a pH of 4 using 10% hydrochloric acid, and extracted with ethyl acetate. The organic layer was washed with brine, and dried with anhydrous magnesium sulfate. The solvent was concentrated under reduced pressure, and the resulting crystals were washed with hexane to obtain 1.6 g of the target compound.

The examples of the compounds according to the present invention, which include those compounds disclosed in the preceding examples, are shown in Table 1. Note that the meaning of the notations and abbreviations in the table is as follows. (& indicates that NMR data is disclosed).
- Me:: methyl
- a1:: 1-imidazolyl
- a2:: 1H-pyrazole-5-yl
- a3:: 1H-pyrazole-4-yl
- a4:: 1-methylpyrazole-5-yl
- a5:: 1-methylpyrazole-3-yl
- a6:: 1-benzylpyrazole-4-yl
- a7:: 2-methyl-1-imidazolyl

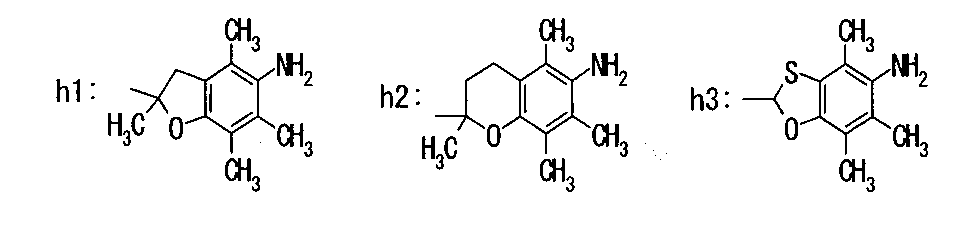

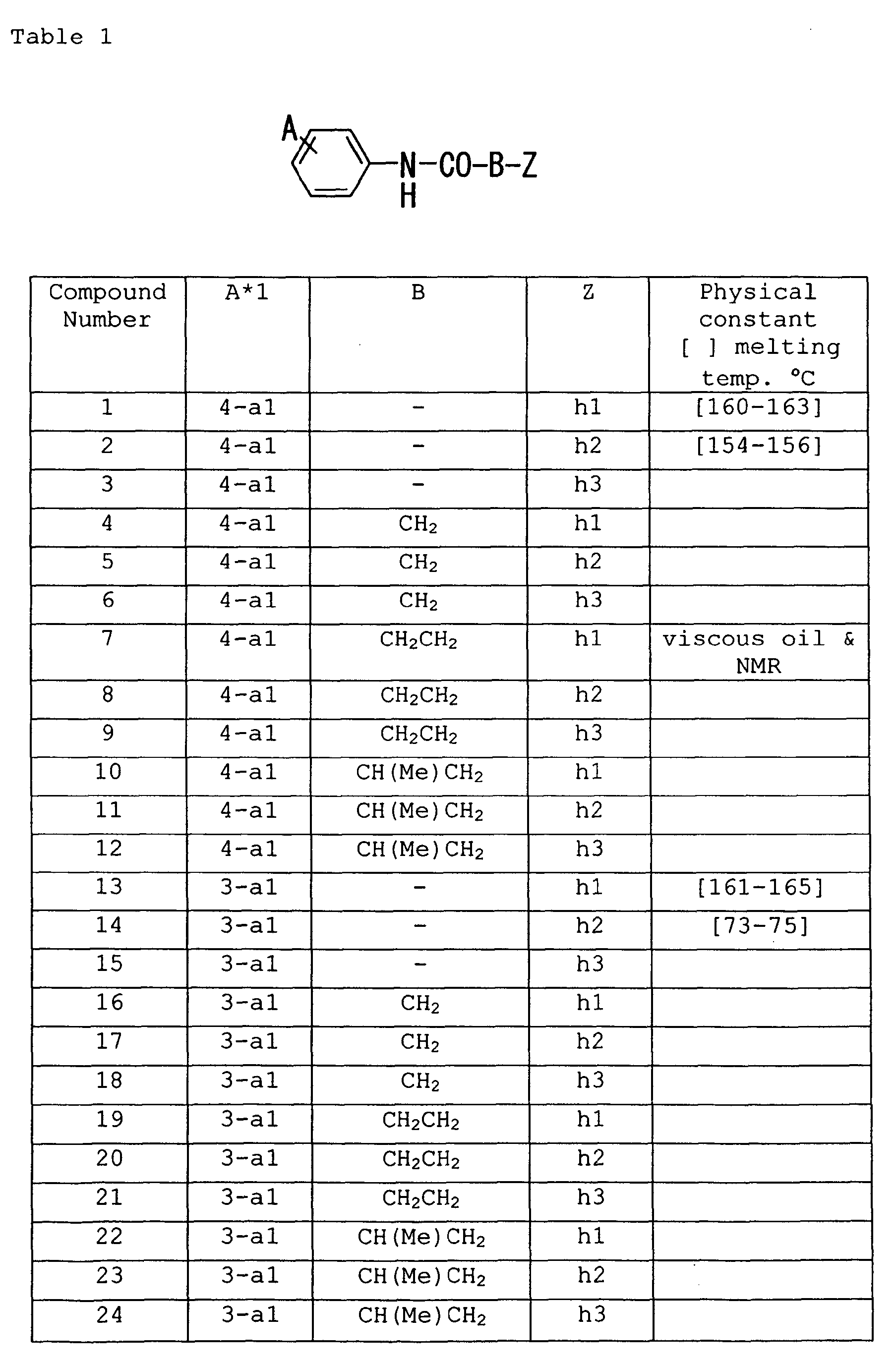

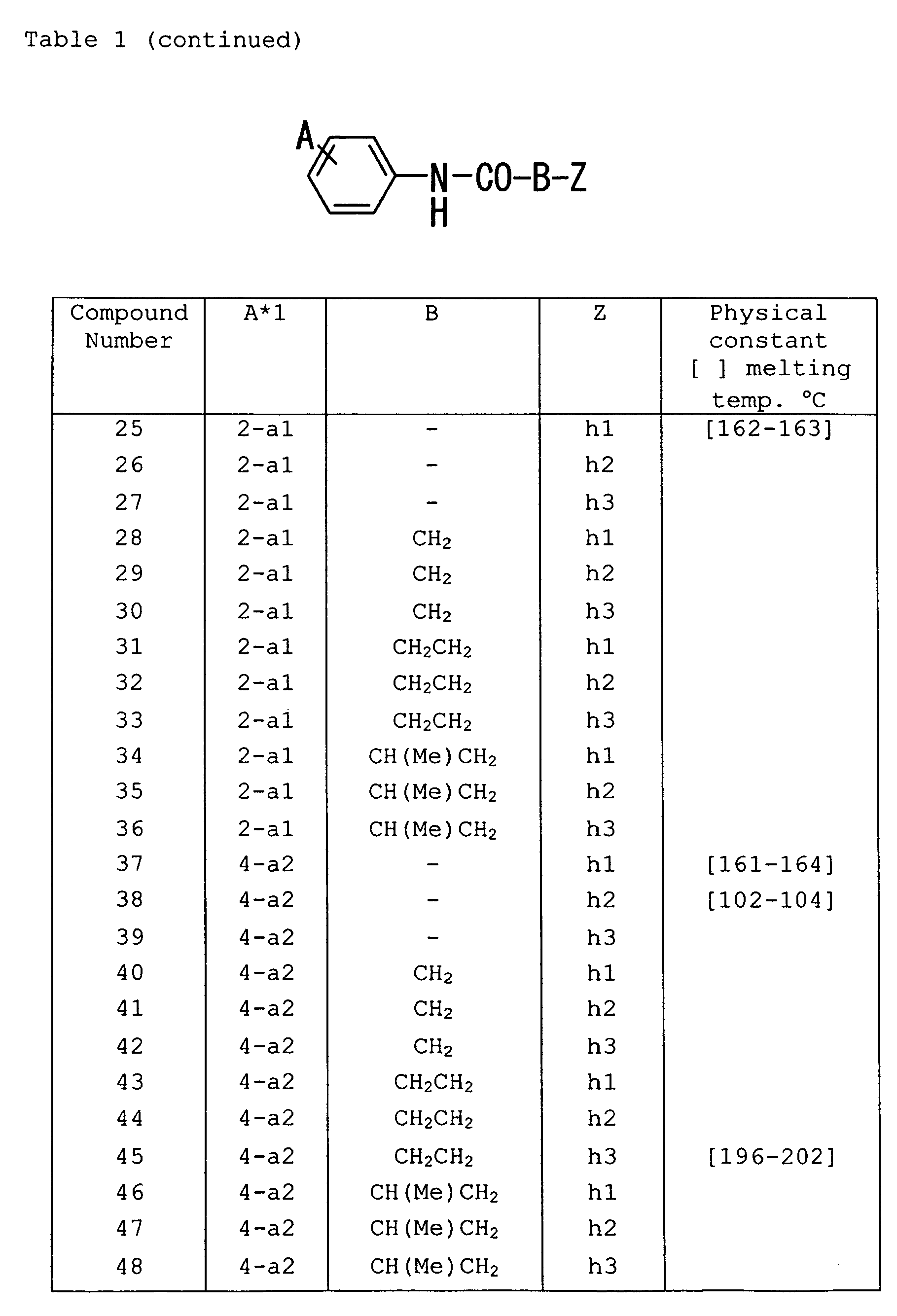

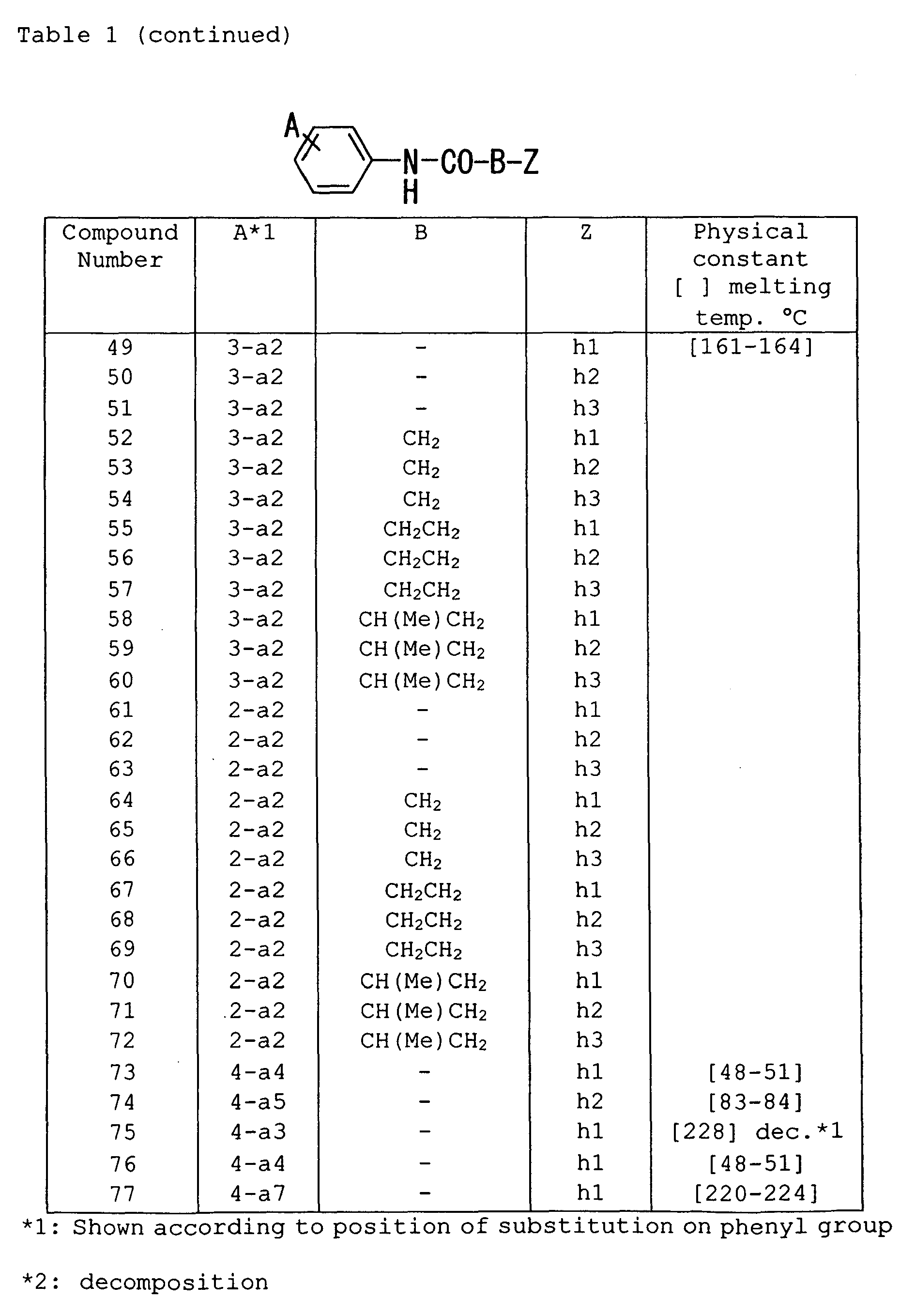

1H-NMR data (heavy chloroform solvent, internal standard TMS) Units is δ. The number inside the parenthesis indicates the proton ratio. Symbols signify as follows: s: singlet, d: doublet, t: triplet, q: quartet, m: multiplet, br: broad, brs: broad singlet.

### Compound Number 7 in Table 1

1.5 (s, 3H), 2.02 (s, 3H), 2.07 (s, 6H), 2.1-2.3 (m, 2H), 2.5 (t, 2H), 3.0 (dd, 2H), 7.2 (s, 1H), 7.25 (s, 1H), 7.27 (s, 1H), 7.3 (d, 2H), 7.6 (m, 3H), 7.8 (s, 1H)

### Example 2:

### Preparation Production

A preparation containing the compound of the present invention was produced according to the method described below.

**Oral Preparation (tablets containing 10 mg of active ingredient):**

| | |
|---|---|
| Compound of present invention | 10 mg |
| Lactose | 81.4 mg |
| Cornstarch | 20 mg |
| Hydroxypropyl cellulose | 4 mg |
| Calcium carboxymethyl cellulose | 4 mg |
| Magnesium stearate | 0.6 mg |
| Total | 120 mg |

50 g of the compound of the present invention, 407 g of lactose and 100 g of cornstarch were uniformly mixed using a fluid bed granulator coating device (manufactured by Okawara MFG. CO., LTD.) to obtain the composition indicated above. 200 g of 10% aqueous hydroxypropyl cellulose solution were then sprayed thereon to produce granules. After drying, the granules were passed through a 20 mesh sieve followed by the addition of 20 g of calcium carboxymethyl cellulose and 3 g of magnesium stearate to obtain tablets containing 120 mg per tablet by using a rotary tablet making machine (manufactured by Hata Iron Works, Ltd.) with a mortar pestle with a dimension of 7 mm x 8.4R.

### Example 3:

### (Antioxidative Action on Lipids In Vitro)

The antioxidative action on lipids in vitro of the compounds according to the present invention was evaluated in accordance with the method of Malvy et al. (Malvy C. et al.,) described in Biochemical and Biophysical Research Communications, 1980, vol. 95, pp. 734 to 737,) by measuring the lipid peroxide activity in a rat brain homogenate. Namely, the rat brain was removed followed by the addition of five volumes of an aqueous solution of phosphate buffered saline (pH 7.4) (hereinafter, abbreviated as PBS) to the brain while cooling with water, homogenizing with a Teflon homogenizer, and centrifuging for 20 minutes at 10,000 g to collect the supernatant as a brain homogenate. 500 µM cysteine, 5 µM ferrous sulfate, and 100 mM KCl were added to the resulting brain homogenate followed by incubating for 30 minutes at 37°C and measuring the malondialdehyde that formed due to decomposition of lipid peroxide using the thiobarbituric acid method. The 50% inhibitory concentrations (hereinafter, abbreviated as IC₅₀) of the compounds according to the present invention were then determined from the measured values. Those results are shown in Table 2. It is apparent that the compounds according to the present invention exhibited antioxidative action on lipids in vitro.

**Table 2**

| Compound No. | 50% inhibitory concentration of anti-lipid peroxide action in vitro (IC₅₀, µM) |
|---|---|
| 1 | 3.3 |
| 2 | 2.9 |
| 14 | 2.5 |
| 37 | 0.80 |
| 49 | 1.0 |
| Control drug 1 | 0.23 |
| Control drug 2 | 0.23 |

### Example 4:

### (Tissue Migration)

Tissue migration of the compounds according to the present invention was evaluated by measuring the antioxidative action on lipids ex vivo. A test compound dissolved or suspended in an aqueous physiological saline solution or aqueous physiological saline solution containing 1% polyethylene hardened castor oil (Nikko Chemicals: Nikkol HCO-60) was intraperitoneally administered to male SD rats (age: 6 weeks) (purchased from Japan SLC) in groups of 3 animals each at a dose of 100 mg/kg. The animals were sacrificed by exsanguination by severing the carotid artery 30 minutes after administration followed by removing the brain, heart and kidneys. The lipid peroxide activity of homogenates of each tissue was measured using the method described in Example 3. The inhibition rates of the test compounds in each tissue were determined from the amounts of lipid peroxide formed in a control group (physiological saline dose group) and test compound groups. Those results are shown in Table 3. On the basis of these results, the compounds of the present invention clearly had a high degree of tissue migration.

**Table 3**

| Compound No. | Inhibition rate of ex vivo anti-lipid peroxide action (%) | | |
|---|---|---|---|
| | Brain | Heart | Kidney |
| 1 | 83 | 80 | 87 |
| 2 | 97 | 69 | 81 |
| 14 | 84 | 84 | 91 |
| 37 | 95 | 87 | 89 |
| 49 | 98 | 87 | 90 |
| Control Drug 1 | 68 | 59 | 75 |
| Control Drug 2 | 45 | 57 | 84 |

### Example 5:

### (Antioxidative Action In Vivo)

The antioxidative action in vivo of the compounds according to the present invention was evaluated according to the method described in the Journal of Medical Chemistry (J. Med. Chem., 1997, vol. 40, pp. 559 to 573) based on the inhibitory effects on abnormal behavior and mortality rate following administration of ferrous chloride into the spinal subarachnoid cavity of mice. Male S1c:ICR mice (age: 5 weeks) (purchased from Japan SLC) were used in groups of 3 to 7 animals each, and were administered 5 µl of aqueous physiological saline solution containing 50 mM ferrous chloride into the spinal column between the 5th and 6th lumbar vertebra. Symptoms were observed for 20 to 60 minutes following administration of ferrous chloride, and scores were determined after 60 minutes based on the symptoms shown in Table 4. The test compounds were dissolved or suspended in an aqueous physiological saline solution or an aqueous physiological saline solution containing 1% polyethylene hardened castor oil (manufactured by Nikko Chemicals CO., LTD. under the name of NIKKOL HCO-60), and administered intraperitoneally or orally 30 minutes before the administration of ferrous chloride. The 50% inhibitory dose (hereinafter, abbreviated as ID₅₀) of each test compound was determined from the score of a control group (physiological saline treated group) and the score of each test compound treated group. Those results are shown in Table 5. It is apparent on the basis of these results that the compounds according to the present invention had the antioxidative action in vivo.

**Table 4**

| Score | Symptoms |
|---|---|
| 0 | Normal |
| 1 | Frequent biting of lower abdomen or end of posterior trunk |
| 2 | Observation of at least one of the following changes: |
| | (1) Frequent biting of posterior trunk while turning around |
| | (2) Hypersensitivity and attacking response to external stimuli |
| | (3) Trembling |
| 3 | Clonic convulsion |
| 4 | Tonic convulsion or paralysis of posterior trunk |
| 5 | Death |

**Table 5**

| Compound No. | Antioxidative action in vivo 50% inhibitory dose (ID₅₀ mg/kg) | |
|---|---|---|
| | Intraperitoneal administration | oral administration |
| 1 | 6.8 | 9.7 |
| 2 | 14 | 18 |
| 14 | 17 | 30 |
| 37 | 12 | 27 |
| 49 | 14 | >30 |
| Control Drug 1 | >30 | >30 |
| Control Drug 2 | 20 | 53 |

Compounds described in International Publication No. WO 00/00650 were used for the control drugs. Control drug 1 is the compound shown below. 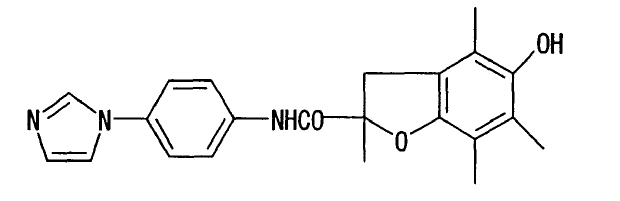 Control drug 2 is the compound shown below.

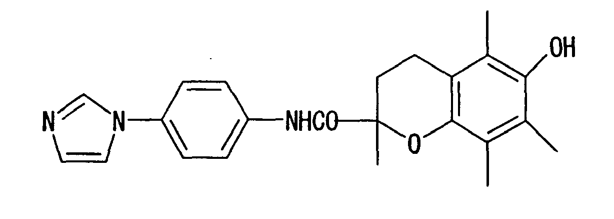

### Example 6:

### (Retina Migration)

The retina migration of the compounds according to the present invention was evaluated. The test compounds dissolved or suspended in 0.1 N aqueous hydrochloric acid solution or 1% polyethylene hardened castor oil (Nikkol HCO-60) solution were orally administered to male SD rats (age: 6 weeks) in groups of three animals each followed by removing both eyes 30 minutes later and separating the retinas while cooling with ice. The retinas were homogenized with a Polytron Microhomogenizer (NS-310E: manufactured by Niti-On Medical and Physical Instruments) in 0.1 M Tris-HCl buffer (pH 7.4) while cooling with ice to prepare 5% homogenates. The homogenates were then auto-oxidized for 1 hour at 37°C, and the amounts of lipid peroxide that formed were quantified using the thiobarbituric acid method (Masugi, et al., Vitamin, 51, 21 to 29, 1977 ) . The dose that resulted in 30% inhibition (ID₃₀) was determined from the inhibition rate at each dose level. Those results are shown in Table 6. It is apparent on the basis of these results that the compounds according to the present invention had action that inhibits the formation of lipid peroxides in the retina ex vivo, and exhibited a high degree of retina migration.

**Table 6**

| Compound No. | Inhibitory effect on formation of lipid peroxides in retina ex vivo 30% inhibitory concentration (ID₃₀ mg/kg, oral administration) |
|---|---|
| 1 | 16 |
| 37 | 5.2 |

### Example 7:

### (Inhibitory Action on 5-Lipoxygenase (5-LO) and 15-Lipoxygenase (15-LO))

5-LO inhibitory activity was measured using a variation of the method of Carter et al . (Carter G. W. et al . , J. Pharmacol. Exp. Ther., 256, 929 to 937, 1991). Namely, after pre-incubating (37°C, 15 minutes) in Hank's solution human peripheral blood mononuclear cells and a test compound dissolved in DMSO (final concentration: 10% ) , 30 µM A23187 was added and the resulting solution was additionally incubated (37°C, 30 minutes). The resulting leucotriene B4 that formed was quantified by enzyme immunoassay, and the 50% formation inhibitory concentration (µM) of the test compound with respect to 5-LO was calculated from that value. Those results are shown in Table 7.

15-LO inhibitory activity was measured using a variation of the method of Auerbach et al. (Auerbach B.J. et al., Anal. Biochem., 201, 375 to 380, 1992). Namely, after pre-incubating (4°C, 15 minutes) in phosphate buffer (pH 7.4) 15-LO obtained from rabbit reticulocytes and a test compound dissolved in DMSO (final concentration: 1%), 256 µM linoleic acid was added and the resulting solution was additionally incubated (4°C, 10 minutes) . The resulting 15-HETE that formed was quantified by spectrophotometry (OD₆₆₀ nm) , and the 50% formation inhibitory concentration (µM) of the test compound with respect to 15-LO was calculated from that value. Those results are shown in Table 7. It is apparent on the basis of these results that the compound of the present invention had inhibitory action on 5-lipoxygenase (5-LO) and 15-lipoxygenase (15-LO).

**Table 7**

| Compound No. | Lipoxygenase inhibitory action 50% inhibitory concentration (IC₅₀ µm) | |
|---|---|---|
| | 5-LO | 15-LO |
| 37 | 0.23 | 2.06 |

### INDUSTRIAL APPLICABILITY

The phenylazole compound of the present invention or the pharmaceutical acceptable salt thereof has antioxidative activity that is effective for the treatment of arteriosclerosis as well as ischemic organ disorders such as myocardial infarction and cerebral stroke, or for the treatment of diseases caused by oxidative cytotoxicity, is able to effectively inhibit retina disorders caused by photooxidation or the like, can be used in the form of a superior antioxidant that contains the phenylazole compound according to the present invention, and is useful as a drug for inhibiting oxidative disorders of the retina that demonstrates few adverse side effects.

## Claims

1. A compound represented by formula (1): 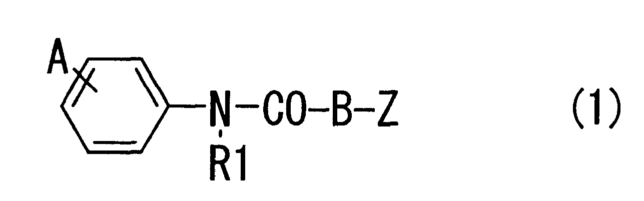 (wherein,
R1 represents a hydrogen atom or a C₁₋₆ alkyl group which may be substituted,
A represents an imidazolyl group or a pyrazolyl group represented by the following formulae: 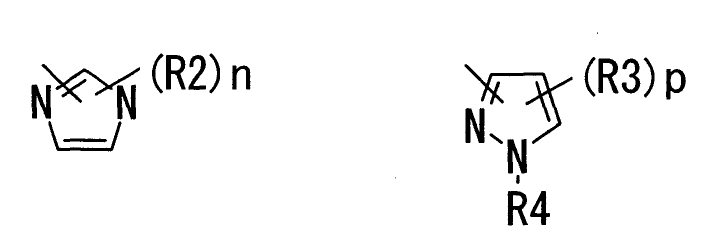
(wherein
R2 and R3 represent a hydrogen atom or a C₁₋₆ alkyl group which may be substituted by G1,
R4 represents a hydrogen atom or a C₁₋₆ alkyl group which may be substituted by G1, a C₁₋₆ alkylcarbonyl group which may be substituted by G1, or a benzoyl group which may be substituted by G1,
n represents 0 or an integer of 1 to 3,
p represents 0 or an integer of 1 or 2, and
R2 and R3 may be identical to each other, or different from each other, when n and p are 2 or more),
B represents a group represented by the following formula: 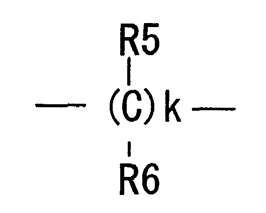
(wherein
R5 and R6 each independently represents a hydrogen atom, a cyano group, a hydroxyl group, a halogen atom, a C₁₋₆ alkyl group, a C₁₋₆ alkoxy group, a C₂₋₆ alkenyl group, a C₂₋₆ alkynyl group, a C₂₋₆ alkenyloxy group, a C₂₋₆ alkynloxy group, a C₁₋₆ acyloxy group, or a C₃₋₆ cycloalkyl group, or a phenyl group which may have a substituent,
k represents 0 or an integer of 1 to 15, and
R5 and R6 may be identical to each other, or different from each other, when k is 2 or more), and
Z represents a chroman-2-yl group which is substituted by G2, a 2, 3-dihydrobenzofuran-2-yl group which is substituted by G2, a thiochroman-2-yl group which is substituted by G2, a 2,3-dihydrobenzothiophene-2-yl group which is substituted by G2, or a 1,3-benzoxathiol-2-yl group which is substituted by G2,
G1 represents a cyano group, a formyl group, a hydroxyl group, an amino group, a dimethylamino group, or a halogen atom,
G2 is represented by the following formula: NHR (wherein R represents a hydrogen atom, a C₁₋₆ alkylcarbonyl group, or a benzoyl group which may have a substituent), or a pharmaceutically acceptable salt thereof.

2. A compound or pharmaceutically acceptable salt according to claim 1, wherein z is a group represented by the following formula (A), (B) or (C): 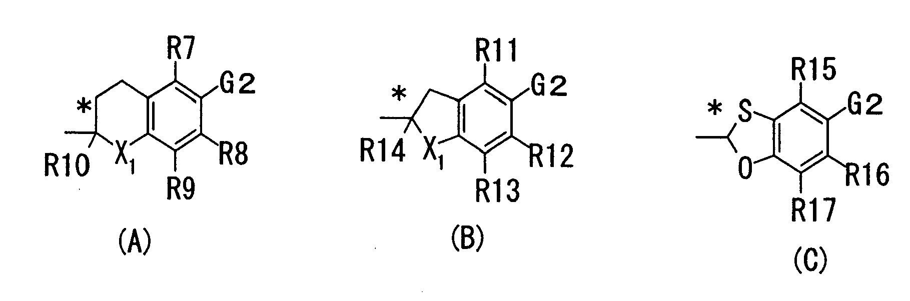 (wherein
* represents an asymmetric carbon atom,
X1 represents an oxygen atom or a sulfur atom,
R7 to R17 each independently represents a hydrogen atom or a C₁₋₆ alkyl group, and
G2 is represented by the following formula: NHR (wherein R represents a hydrogen atom, a C₁₋₆ alkylcarbonyl group, or a benzoyl group which may have a substituent)).

3. A compound or pharmaceutically acceptable salt according to claim 1 or 2, wherein A is 1-imidazolyl or 1-H-pyrazole-5-yl which is substituted at the fourth position on the benzene ring.

4. A production process of a compound represented by formula (1) : 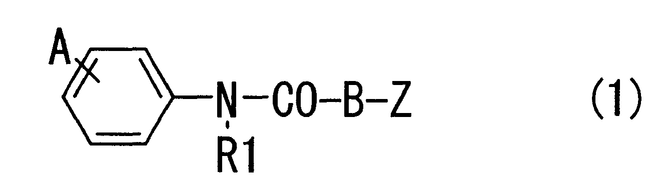 (wherein,
R1 represents a hydrogen atom or a C₁₋₆ alkyl group which may be substituted,
A represents an imidazolyl group or a pyrazolyl group represented by the following formulae: 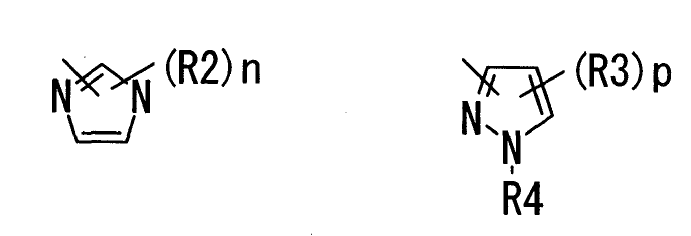
(wherein
R2 and R3 represent a hydrogen atom or a C₁₋₆ alkyl group which may be substituted by G1,
R4 represents a hydrogen atom or a C₁₋₆ alkyl group which may be substituted by G1, a C₁₋₆ alkylcarbonyl group which may be substituted by G1, or a benzoyl group which may be substituted by G1,
n represents 0 or an integer of 1 to 3,
p represents 0 or an integer of 1 or 2, and
R2 and R3 may be identical to each other, or different from each other, when n and p are 2 or more),
B represents a group represented by the following formula: 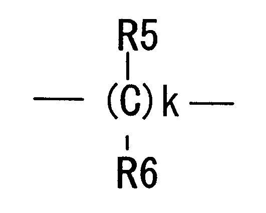
(wherein
R5 and R6 each independently represents a hydrogen atom, a cyano group, a hydroxyl group, a halogen atom, a C₁₋₆ alkyl group, a C₁₋₆ alkoxy group, a C₂₋₆ alkenyl group, a C₂₋₆ alkynyl group, a C₂₋₆ alkenyloxy group, a C₂₋₆ alkynloxy group, a C₁₋₆ acyloxy group, or a C₃₋₆ cycloalkyl group, or a phenyl group which may have a substituent,
k represents 0 or an integer of 1 to 15, and
R5 and R6 may be identical to each other, or different from each other, when k is 2 or more),
Z represents a chroman-2-yl group which is substituted by G2, a 2, 3-dihydrobenzofuran-2-yl group which is substituted by G2, a thiochroman-2-yl group which is substituted by G2, a 2,3-dihydrobenzothiophene-2-yl group which is substituted by G2, or a 1,3-benzoxathiol-2-yl group which is substituted by G2,
G1 represents a cyano group, a formyl group, a hydroxyl group, an amino group, a dimethylamino group, or a halogen atom, and
G2 is represented by the following formula: NHR (wherein R represents a hydrogen atom, a C₁₋₆ alkylcarbonyl group, or a benzoyl group which may have a substituent),
comprising:
a step 1 in which a compound represented by the following formula (1') 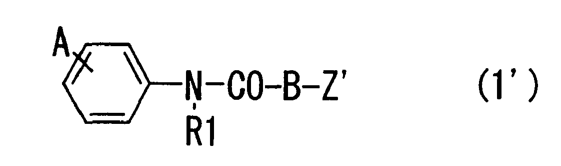 (wherein
R1 represents a hydrogen atom or a C₁₋₆ alkyl group which may be substituted,
A represents an imidazolyl group or a pyrazolyl group represented by the following formulae: 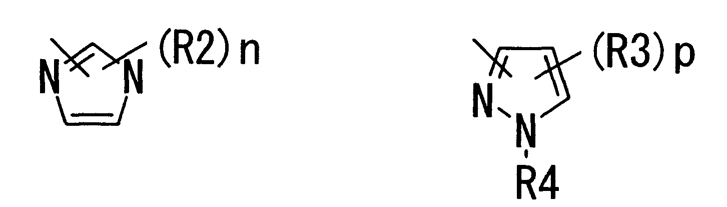
(wherein
R2 and R3 represent a hydrogen atom or a C₁₋₆ alkyl group which may be substituted by G1,
R4 represents a hydrogen atom or a C₁₋₆ alkyl group which may be substituted by G1, a C₁₋₆ alkylcarbonyl group which may be substituted by G1, or a benzoyl group which may be substituted by G1,
n represents 0 or an integer of 1 to 3,
p represents 0 or an integer of 1 or 2, and
R2 and R3 may be identical to each other, or different from each other, when n and p are 2 or more)),
B represents a group represented by the following formula: 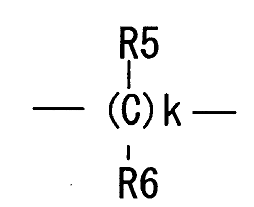
(wherein
R5 and R6 each independently represents a hydrogen atom, a cyano group, a hydroxyl group, a halogen atom, a C₁₋₆ alkyl group, a C₁₋₆ alkoxy group, a C₂₋₆ alkenyl group, a C₂₋₆ alkynyl group, a C₂₋₆ alkenyloxy group, a C₂₋₆ alkynloxy group, a C₁₋₆ acyloxy group, or a C₃₋₆ cycloalkyl group, or a phenyl group which may have a substituent,
k represents 0 or an integer of 1 to 15, and
R5 and R6 may be identical to each other, or different from each other, when k is 2 or more), and
Z' is represented by the following formula (A)', (B)', or (C)': 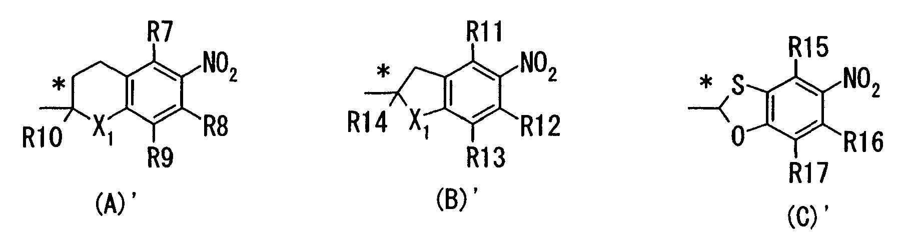 (wherein
* represents an asymmetric carbon atom,
X1 represents an oxygen atom or a sulfur atom,
R7 to R17 each independently represents a hydrogen atom or a C₁₋₆ alkyl group, and
G2 is represented by the following formula: NHR (wherein R represents a hydrogen atom, a C₁₋₆ alkylcarbonyl group, or a benzoyl group which may have a substituent)) is produced by reacting an amine compound represented by formula (2) : 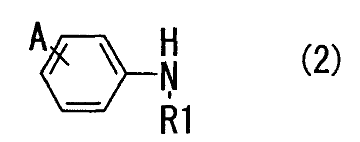
(wherein
R1 represents a hydrogen atom or a C₁₋₆ alkyl group which may be substituted, and
A represents an imidazolyl group or a pyrazolyl group represented by the following formulae: 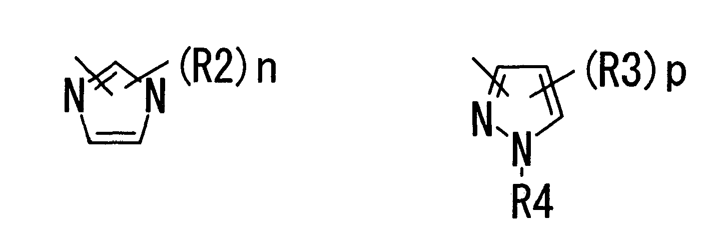
(wherein
R2 and R3 represent a hydrogen atom or a C₁₋₆ alkyl group which may be substituted by G1,
R4 represents a hydrogen atom or a C₁₋₆ alkyl group which may be substituted by G1, a C₁₋₆ alkylcarbonyl group which may be substituted by G1, or a benzoyl group which may be substituted by G1,
n represents 0 or an integer of 1 to 3,
p represents 0 or an integer of 1 or 2, and
R2 and R3 may be identical to each other, or different from each other, when n and p are 2 or more)) with a compound represented by the following formula (3):
YOC-B-Z' (3)
(wherein
Y represents a hydroxyl group or a halogen atom,
B represents a group represented by the following formula: 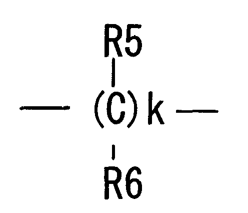 (wherein
R5 and R6 each independently represents a hydrogen atom, a cyano group, a hydroxyl group, a halogen atom, a C₁₋₆ alkyl group, a C₁₋₆ alkoxy group, a C₂₋₆ alkenyl group, a C₂₋₆ alkynyl group, a C₂₋₆ alkenyloxy group, a C₂₋₆ alkynloxy group, a C₁₋₆ acyloxy group, or a C₃₋₆ cycloalkyl group, or a phenyl group which may have a substituent,
k represents 0 or an integer of 1 to 15, and
R5 and R6 may be identical to each other, or different from each other, when k is 2 or more) and
Z' is represented by the following formula (A)', (B)', or (C)': 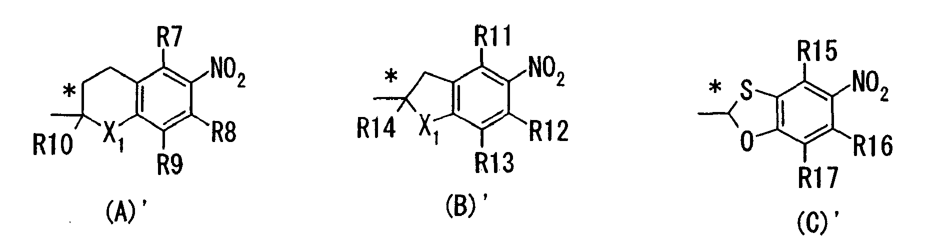
(wherein
* represents an asymmetric carbon atom,
X1 represents an oxygen atom or a sulfur atom,
R7 to R17 each independently represents a hydrogen atom or a C₁₋₆ alkyl group, and
G2 is represented by the following formula: NHR
(wherein R represents a hydrogen atom, a C₁₋₆ alkylcarbonyl group, or a benzoyl group which may have a substituent)); and
a step 2 in which the nitro compound produced in the step 1 is converted to an amino group using a reducing agent.

5. An antioxidant comprising as its active ingredient at least one compound represented by formula (1): 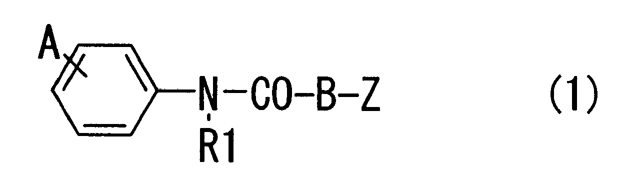 (wherein
R1 represents a hydrogen atom or a C₁₋₆ alkyl group which may be substituted,
A represents an imidazolyl group or a pyrazolyl group represented by the following formulae: 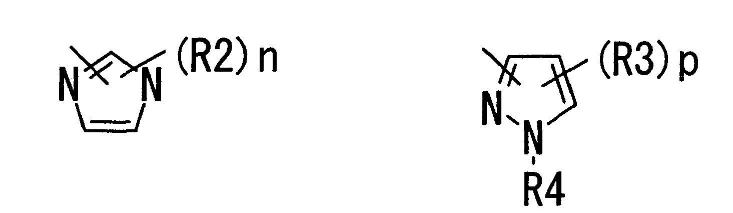
(wherein
R2 and R3 represent a hydrogen atom or a C₁₋₆ alkyl group which may be substituted by G1,
R4 represents a hydrogen atom or a C₁₋₆ alkyl group which may be substituted by G1, a C₁₋₆ alkylcarbonyl group which may be substituted by G1, or a benzoyl group which may be substituted by G1,
n represents 0 or an integer of 1 to 3,
p represents 0 or an integer of 1 or 2, and
R2 and R3 may be identical to each other, or different from each other, when n and p are 2 or more)),
B represents a group represented by the following formula: 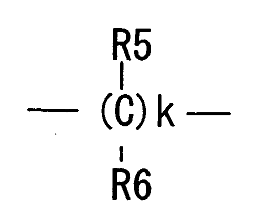
(wherein
R5 and R6 each independently represents a hydrogen atom, a cyano group, a hydroxyl group, a halogen atom, a C₁₋₆ alkyl group, a C₁₋₆ alkoxy group, a C₂₋₆ alkenyl group, a C₂₋₆ alkynyl group, a C₂₋₆ alkenyloxy group, a C₂₋₆ alkynloxy group, a C₁₋₆ acyloxy group, or a C₃₋₆ cycloalkyl group, or a phenyl group which may have a substituent,
k represents 0 or an integer of 1 to 15, and
R5 and R6 may be identical to each other, or different from each other, when k is 2 or more),
Z represents a chroman-2-yl group which is substituted by G2, a 2,3-dihydrobenzofuran-2-yl group which is substituted by G2, a thiochroman-2-yl group which is substituted by G2, a 2,3-dihydrobenzothiophene-2-yl group which is substituted by G2, or a 1,3-benzoxathiol-2-yl group which is substituted by G2,
G1 represents a cyano group, a formyl group, a hydroxyl group, an amino group, a dimethylamino group, or a halogen atom, and
G2 is represented by the following formula: NHR (wherein R represents a hydrogen atom, a C₁₋₆ alkylcarbonyl group, or a benzoyl group which may have a substituent) or a pharmaceutically acceptable salt thereof.

6. An antioxidant according to claim 5, wherein in formula (1) z is represented by the following formula (A) , (B) , or (C) : 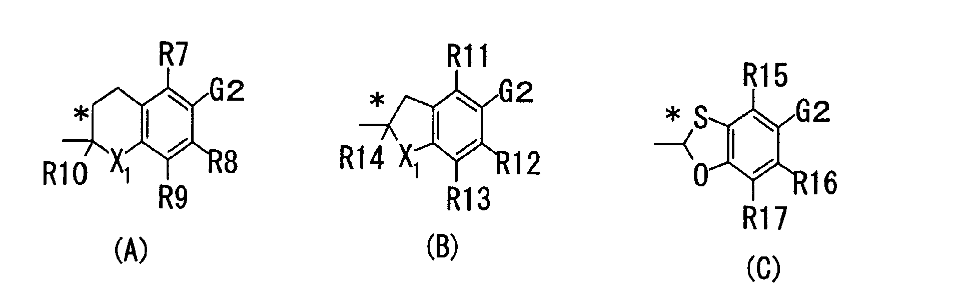 (wherein
* represents an asymmetric carbon atom,
X1 represents an oxygen atom or a sulfur atom,
R7 to R17 each independently represents a hydrogen atom or a C₁₋₆ alkyl group, and
G2 is represented by the following formula: NHR
(wherein R represents a hydrogen atom, a C₁₋₆ alkylcarbonyl group, or a benzoyl group which may have a substituent)).

7. A kidney disease, cerebrovascular or cardiovascular disease treatment agent **characterized by** comprising the antioxidant according to claim 6.

8. A cerebral infarction treatment agent **characterized by** comprising the antioxidant according to claim 6.

9. A retinal oxidation disorder inhibitor **characterized by** comprising the antioxidant according to claim 6.

10. A retinal oxidation disorder inhibitor according to claim 9 for age-related macular degeneration or diabetic retinopathy.

11. A lipoxygenase inhibitor **characterized by** comprising the antioxidant according to claim 6.

12. A 20-hydroxyeicosatetraenoic acid (20-HETE) synthase inhibitor **characterized by** comprising the antioxidant according to claim 6.
